# EUROPEAN PATENT SPECIFICATION

(11) **EP 1 815 255 B1**
(45) Date of publication and mention of the grant of the patent: **22.04.2009**
(21) Application number: 05811604.7
(22) Date of filing: 25.11.2005
(51) Int. Cl.: G01N 33/68

(54) **USE OF EPH RECEPTOR INHIBITORS FOR THE TREATMENT OF NEURODEGENERATIVE DISEASES**
VERWENDUNG VON EPH-REZEPTOR-INHIBITOREN ZUR BEHANDLUNG NEURODEGENERATIVER KRANKHEITEN
UTILISATION D'INHIBITEURS DU RECEPTEUR EPH POUR LE TRAITEMENT DE MALADIES NEURODEGENERATIVES

(30) Priority: 25.11.2004 EP 04028046; 26.11.2004 US 630592 P
(43) Date of publication of application: 08.08.2007
(73) Proprietor: CELLZOME AG, 69117 Heidelberg (DE)
(72) Inventor: HOPF, Carsten, 68165 Mannheim (DE); DREWES, Gerard, 69121 Heidelberg (DE)
(74) Representative: Huhn, Michael
(86) International application number: PCT/EP2005/012649
(87) International publication number: WO 2006/056467

(56) References cited:
- WO-A-00/38675
- WO-A-03/104222
- WO-A-20/04028551
- US-A1- 2004 028 673
- PATEL S ET AL: "Apo and Inhibitor Complex Structures of BACE (beta-secretase)" JOURNAL OF MOLECULAR BIOLOGY, LONDON, GB, vol. 343, no. 2, 15 October 2004 (2004-10-15), pages 407-416, XP004608978 ISSN: 0022-2836

## Description

The present invention relates to methods for the screening of gamma secretase inhibitors.

Alzheimer's disease is a chronic condition that affects millions of individuals worldwide.

The brains of sufferers of Alzheimer's disease show a characteristic pathology of prominent neuropathologic lesions, such as the initially intracellular neurofibrillary tangles (NFTs), and the extracellular amyloid-rich senile plaques. These lesions are associated with massive loss of populations of CNS neurons and their progression accompanies the clinical dementia associated with AD. The major components of amyloid plaques are the amyloid beta (A-beta, Abeta or Aß) peptides of various lengths. A variant thereof, which is the Aß1-42-peptide (Abeta-42) is the major causative agent for amyloid formation. Amyloid beta is the proteolytic product of a precursor protein, beta amyloid precursor protein (beta-APP or APP). APP is a type-I trans-membrane protein which is sequentially cleaved by several different membrane-associated proteases. The first cleavage of APP occurs by one of two proteases, alpha-secretase or beta-secretase. Alpha secretase is a metalloprotease whose activity is most likely to be provided by one or a combination of the proteins ADAM10 and ADAM17. Cleavage by alpha-secretase precludes formation of amyloid peptides and is thus referred to as non-amyloidogenic. In contrast, cleavage of APP by beta-secretase is a prerequisite for subsequent formation of amyloid peptides. This secretase, also called BACE1 (beta-site APP-cleaving enzyme), is a type-I transmembrane protein containing an aspartyl protease activity (described in detail below).

The beta-secretase (BACE) activity cleaves APP in the ectodomain, resulting in shedding of secreted, soluble APPb, and in a 99-residue C-terminal transmembrane fragment (APP-C99). Vassar et al. (Science 286, 735-741) cloned a transmembrane aspartic protease that had the characteristics of the postulated beta-secretase of APP, which they termed BACE1. Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids (aa) containing a 21-aa signal peptide followed by a proprotein domain spanning aa 22 to 45. There are alternatively spliced forms, BACE-I-457 and BACE-I-476. The extracellular domain of the mature protein is followed by one predicted transmembrane domain and a short cytosolic C-terminal tail of 24 aa. BACE1 is predicted to be a type 1 transmembrane protein with the active site on the luminal side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (J. Biol. Chem. 279, 10542-10550). To date, no BACE1 interacting proteins with regulatory or modulatory functions have been described. Similarly, no proteins that modulate signal transduction events or metabolism and thereby regulate or modulate BACE1 activity have been characterized.

The APP fragment generated by BACE1 cleavage, APP-C99, is a substrate for the gamma-secretase activity, which cleaves APP-C99 within the plane of the membrane into an A-beta peptide (such as the amyloidogenic Aß1-42 peptide), and into a C-terminal fragment termed APP intracellular domain (AICD) (Annu Rev Cell Dev Biol 19, 25-51). The gamma-secretase activity resides within a multiprotein complex with at least four distinct subunits. The first subunit to be discovered was presenilin (Proc Natl Acad Sci USA 94, 8208-13). Other known protein components of the gamma-secretase complex are Pen-2, Nicastrin and Aph-1 a.

Despite recent progress in delineating molecular events underlying the etiology of Alzheimer's disease, no disease-modifying therapies have been developed so far. To this end, the industry has struggled to identify suitable lead compounds for inhibition of BACE1. Moreover, it has been recognized that a growing number of alternative substrates of gamma-secretase exist, most notably the Notch protein. Consequently, inhibition of gamma-secretase is likely to cause mechanism-based side effects. Current top drugs (e.g. Aricept®/donepezil) attempt to achieve a temporary improvement of cognitive functions by inhibiting acetylcholinesterase, which results in increased levels of the neurotransmitter acetylcholine in the brain. These therapies are not suitable for later stages of the disease, they do not treat the underlying disease pathology, and they do not halt disease progression.

Thus, there is an unmet need for the identification of novel targets allowing novel molecular strategies for the treatment of Alzheimer's disease. In addition, there is a strong need for novel therapeutic compounds modifying the aformentioned molecular processes by targeting said novel targets.

In a first aspect, the invention provides a method for the identification of a compound modulating, preferably inhibiting gamma secretase and/or beta secretase activity, comprising the steps of:
a) identifying an Eph receptor modulator, preferably an Eph receptor inhibitor, and
b) determining whether the Eph receptor modulator of step a) is capable of modulating, preferably inhibiting gamma secretase and/or beta secretase activity.

In the context of the present invention, it has been surprisingly found that Eph receptors are involved in the formation of Abeta 42 peptides. Furthermore, it has been surprisingly found that the compounds disclosed in US 2004/0028673A1 and Netzer WJ et al., PNAS 100:12444-12449 are modulators of the activity of Eph receptors. These findings enable the use of Eph receptors in the identification of compounds modulating Eph activity and, thereby, modulating the Abeta 42 production.

Erythropoietin-producing hepatocellular (Eph) receptors are the largest subfamily of receptor tyrosine kinases (RTK). They are subdivided into two classes based on sequence conservation and affinity for their endogenous ligands, ephrins. Ten EphA RTKs (EphA1-EphA10) are activated by six A-ephrins (ephrinsA1-ephrinA6), characterized by the presence of a GPI anchor, whilst six EphB receptors (EphB1-EphB6) predominantly interact with three transmembrane B-ephrins (ephrinB1-ephrinB3).

Eph receptors and ephrin ligands are both membrane-bound and in most cases expressed by adjacent cells (i.e. in "trans"), their interactions occur at points of cell-cell contact. Ligand-receptor binding activates the kinase domain and induces poly-phosphorylation of Eph receptor intracellular domain and signalling. A unique bidirectional signalling cascade can result from ephrinB-Eph interactions, in which a second signal is triggered by phosphorylation of the ephrin intracellular domain (Palmer A, Klein R (2003) Multiple roles of ephrins in morphogenesis, neuronal networking, and brain function. Genes Dev. 17(12):1429.50).

Signal specificity of signal transduction processes on a molecular level as well as biological processes on a systems level is controlled by: tissue/cell type-specific expression; developmentally controlled expression; distinct but overlapping ligand specificity; distinct but overlapping recruitment of adapters and other signal transduction mediators;

Signal diversity is increased by receptor-ligand promiscuity; receptor transphosphorylation; cross-talk with other families of RTKs; positioning of ligand and receptor either in cis or in trans (Yin Y, Yamashita Y, Noda H, Okafuji T, Go MJ, Tanaka H (2004) EphA receptor tyrosine kinases interact with co-expressed ephrin-A ligands in cis. Neurosci Res. 48(3):285-96).

According to the present invention, the expression "Eph receptor" does not only mean the protein itself as published, but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X. SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

The same applies also to all other proteins named in the present invention. Therefore, a name of given protein or nucleic acid does not only refer to the protein or nucleic acid itself, but also to its functionally active derivative, or to a functionally active fragment thereof, or a homologue thereof, or a variant encoded by a nucleic acid that hybridizes to the nucleic acid encoding said protein under low stringency conditions, preferably under the conditions as mentioned above.

Although kinase-independent functions of Eph receptors have been described, most of functional roles have been attributed to their endogenous kinase activity. For example, Miao et al., (Miao H, Wei BR, Peehl DM, Li Q, Alexandrou T, Schelling JR, Rhim JS, Sedor JR, Bumett E, Wang B (2001) Activation of EphA receptor tyrosine kinase inhibits the Ras/MAPK pathway. Nat Cell Biol. 3(5):527-30) observed that integrin-mediated cell adhesion but not directional cell migration requires catalytic activity of EphB3 receptor tyrosine kinase - implicating a distinct signaling pathway to Rho GTPases in the latter.

In the case of other proteins, the term "functionally active" as used herein refers to a polypeptide, namely a fragment or derivative, having structural, regulatory, or biochemical functions of the protein according to the embodiment of which this polypeptide, namely fragment or derivative is related to.

According to the present invention, the term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, stabilize, inhibit, suppress or destabilize the function of other molecules, stability, ability to localize to certain subcellular locations.

According to the present invention, the terms "derivatives" or "analogs of component proteins" or "variants" as used herein preferably include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions. It means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by suitable available in vitro assays as provided in the invention.

The term "fragment" as used herein refers to a polypeptide of at least 10, 20, 30, 40 or 50 amino acids of the component protein according to the embodiment. In specific embodiments, such fragments are not larger than 35,100 or 200 amino acids.

The term "gene" as used herein refers to a nucleic acid comprising an open reading frame encoding a polypeptide of, if not stated otherwise, the present invention, including both exon and optionally intron sequences.

The terms " homologue" or "homologous gene products" as used herein mean a protein in another species, preferably mammals, which performs the same biological function as the a protein component further described herein. Such homologues are also termed "orthologous gene products". The algorithm for the detection of orthologue gene pairs from humans and mammalians or other species uses the whole genome of these organisms. First, pair wise best hits are retrieved, using a full Smith-Waterman alignment of predicted proteins. To further improve reliability, these pairs are clustered with pair wise best hits involving Drosophila melanogaster and C. elegans proteins. Such analysis is given, e.g., in Nature, 2001, 409:860-921. The homologues of the proteins according to the invention can either be isolated based on the sequence homology of the genes encoding the proteins provided herein to the genes of other species by cloning the respective gene applying conventional technology and expressing the protein from such gene, or by isolating proteins of the other species by isolating the analogous proteins according to the methods provided herein or to other suitable methods commonly known in the art.

Ephs and ephrins regulate various processes during development (Wilkinson DG (2001) Multiple roles of EPH receptors and ephrins in neural development. Nat Rev Neurosci. 2(3):155-64.) including cell migration, repulsion, adhesion or attachment to the extracellular matrix. For instance, in mice lacking EphB2 and EphB3, many hippocampal axons remain in bundles. This phenotype ("axon fasciculation") was also observed in mice in which the cytoplasmic domain of EphB2 was specifically deleted (Chen ZY, Sun C, Reuhl K, Bergemann A, Henkemeyer M, Zhou R (2004) Abnormal hippocampal axon bundling in EphB receptor mutant mice. J Neurosci. 24(10):2366-74) - confirming the notion that Eph kinases have kinase-independent as well as kinase-dependent functions.

As another example, in the small intestine, precursors of absorptive, enteroendocrine, and goblet cells migrate toward the villus while Paneth cells occupy the bottom of the crypts. Catenin and TCF have been shown to inversely control the expression of the EphB2/EphB3 receptors and their ligand ephrin-B1 in colorectal cancer and along the crypt-villus axis. In EphB2/EphB3 null mice, the proliferative and differentiated populations intermingle of various intestinal cells intermingle and are not allocated correctly within the intestinal epithelium (Battle E, Henderson JT, Beghtel H, van den Bom MM, Sancho E, Huls G, Meeldijk J, Robertson J, van de Wetering M, Pawson T, Clevers H (2002) Beta-catenin and TCF mediate cell positioning in the intestinal epithelium by controlling the expression of EphB/ephrinB. Cell 111 (2):251-63.).

Overexpression or overactivation of certain Eph RTKs have been implicated in various cancers and are thought to be important in angiogenesis. Aside from important functions during development or in cancer, the roles of Eph kinases in adult animals are less well characterized. At least four Eph kinase subtypes have been identified in rodent hippocampus, EphB1, B2, B3 and A4. In-vitro studies in slice preparations suggest that trans-synaptic interactions between postsynaptic EphB receptors and presynaptic B-ephrins be necessary for the induction of NMDA receptor-independent mossy fiber LTP (Contractor A, Rogers C, Maron C, Henkemeyer M, Swanson GT, Heinemann SF (2002) Trans-synaptic Eph receptor-ephrin signaling in hippocampal mossy fiber LTP. Science 296(5574):1864-9). As for NMDA receptor-dependent LTP at CA1 and dentate gyrus synapses, mice lacking only the intracellular domain of EphB2 appear normal, whereas animals carrying a deletion of the entire receptor displayed reduced (protein synthesis-dependent) LTP and alterations in synaptic depression. A mechanism whereby EphBs modulate NMDA receptor function has been proposed (Takasu MA, Dalva MB, Zigmond RE, Greenberg ME (2002) Modulation of NMDA receptor-dependent calcium influx and gene expression through EphB receptors. Science 295(5554):491-5.).

As another example of Eph receptor function in vivo, transgenic mice over-expressing EphB4 in the kidney have been shown to develop abnormal glomerular structures reminiscent of aglomerular vascular shunts, a human degenerative glomerulopathy of unknown aetiology (Andres AC, Munarini N, Djonov V, Bruneau S, Zuercher G, Loercher S, Rohrbach V, Ziemiecki A (2003) EphB4 receptor tyrosine kinase transgenic mice develop glomerulopathies reminiscent of aglomerular vascular shunts. Mech Dev. 120(4):511-6).

In the context of the present invention, a "Eph receptor modulator" is a molecule which modulates Eph receptor activity. This can e.g. be the tyrosine kinase activity of the Eph receptor.

Throughout the invention, the term "modulating the activity of gamma secretase and/or beta secretase" includes that the activity of the enzyme is modulated directly or indirectly. That means that the Eph receptor modulator may either bind also directly to either of these enzymes or, more preferred, may exert an influence on the Eph receptor which in turn, e.g. by protein-protein interactions or by signal transduction or via small metabolites, modulates the activity of either of these enzymes.

Throughout the invention, it is preferred that the beta secretase modulator inhibits the activity of beta secretase either completely or partially. Throughout the invention, the most preferred functional consequence of a Eph receptor modulator is a reduction in Abeta-42 generation.

In the context of the present invention, "modulating the activity of gamma secretase and/or beta secretase" means that the activity is reduced in that less or no product is formed, most preferably that less or no Abeta-42 is formed, (partial or complete inhibition) or that the respective enzyme produces a different product (in the case of gamma-secretase e.g. Abeta-38 or other Abeta peptide species of shorter amino acid sequence - instead of Abeta-42) or that the relative quantities of the products are different (in the case of gamma-secretase e.g. the ratio of Abeta-40 to Abeta-42 is changed preferably increased). Furthermore, it is included that the modulator modulates either gamma secretase or beta-secretase or the activity of both enzymes.

With respect to the modulator of gamma secretase activity, it is preferred that this modulator inhibits gamma secretase activity. However, it is also preferred that the activity of gamma secretase is shifted in a way that the total amount of Abeta peptide species is unchanged but that more Abeta-38 is produced instead of Abeta-42.

Gamma secretase activity can e.g. measured by determining APP processing, e.g. by determining levels of Abeta peptide species produced, most importantly levels of Abeta-42 (see Example-section, infra).

The invention thus provides a screening assay for compounds modulating gamma secretase and/or beta secretase activity, i.e. the production of Abeta peptides, wherein the compounds modulate Eph receptor activity.

The method of the invention is preferably performed in the context of a high throughput assay. Such assays are known to the person skilled in the art.

Test or candidate molecules to be screened can be provided as mixtures of a limited number of specified compounds, or as compound libraries, peptide libraries. Agents/molecules to be screened may also include all forms of antisera, antisense nucleic acids, etc., that can modulate protein activity or formation. Exemplary candidate molecules and libraries for screening are set forth below.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with an Eph receptor immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

Methods for screening may involve labeling the proteins with radioligands (e.g., ¹²⁵I or ³H), magnetic ligands (e.g., paramagnetic beads covalently attached to photobiotin acetate), fluorescent ligands (e.g., fluorescein or rhodamine), or enzyme ligands (e.g., luciferase or beta-galactosidase). The reactants that bind in solution can then be isolated by one of many techniques known in the art, including but not restricted to, co-immunoprecipitation of the labeled protein moiety using antisera against the unlabeled binding partner (or labeled binding partner with a distinguishable marker from that used on the second labeled protein moiety), immunoaffinity chromatography, size exclusion chromatography, and gradient density centrifugation. In a preferred embodiment, the labeled binding partner is a small fragment or peptidomimetic that is not retained by a commercially available filter. Upon binding, the labeled species is then unable to pass through the filter, providing for a simple assay of complex formation.

Methods commonly known in the art are used to label the protein. Suitable labeling methods include, but are not limited to, radiolabeling by incorporation of radiolabeled amino acids, e.g., ³H-leucine or ³⁵S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where a protein is immobilized, e.g., as described infra, the free species is labeled.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular complexes can be assayed using routine protein binding assays to determine optimal binding conditions for reproducible binding.

In general, the physical parameters of complex formation can be analyzed by quantification of complex formation using assay methods specific for the label used, e.g., liquid scintillation counting for radioactivity detection, enzyme activity for enzyme-labeled moieties, etc. The reaction results are then analyzed utilizing Scatchard analysis, Hill analysis, and other methods commonly known in the arts (see, e.g., Proteins, Structures, and Molecular Principles, 2nd Edition (1993) Creighton, Ed., W.H. Freeman and Company, New York).

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow complex formation.

After binding is performed, unbound, labeled protein is removed in the supernatant, and the immobilized protein retaining any bound, labeled protein is washed extensively. The amount of bound label is then quantified using standard methods in the art to detect the label as described, supra.

In another specific embodiments screening for modulators of the proteins as provided herein can be carried out by attaching those and/or the antibodies as provided herein to a solid carrier.

The preparation of such an array containing different types of proteins, including antibodies) is well known in the art and is apparent to a person skilled in the art (see e.g. Ekins et al., 1989, J. Pharm. Biomed. Anal. 7:155-168; Mitchell et al. 2002, Nature Biotechnol. 20:225-229; Petricoin et al., 2002, Lancet 359:572-577; Templin et al., 2001, Trends Biotechnol. 20:160-166; Wilson and Nock, 2001, Curr. Opin. Chem. Biol. 6:81-85; Lee et al., 2002 Science 295:1702-1705; MacBeath and Schreiber, 2000, Science 289:1760; Blawas and Reichert, 1998, Biomaterials 19:595; Kane et al., 1999, Biomaterials 20:2363; Chen et al., 1997, Science 276:1425; Vaugham et al., 1996, Nature Biotechnol. 14:309-314; Mahler et al., 1997, Immunotechnology 3:31-43; Roberts et al., 1999, Curr. Opin. Chem. Biol. 3:268-273; Nord et al., 1997, Nature Biotechnol. 15:772-777; Nord et al., 2001, Eur. J. Biochem. 268:4269-4277; Brody and Gold, 2000, Rev. Mol. Biotechnol. 74:5-13; Karlstroem and Nygren, 2001, Anal. Biochem. 295:22-30; Nelson et al., 2000, Electrophoresis 21:1155-1163; Honore et al., 2001, Expert Rev. Mol. Diagn. 3:265-274; Albala, 2001, Expert Rev. Mol. Diagn. 2:145-152, Figeys and Pinto, 2001, Electrophoresis 2:208-216 and references in the publications listed here).

Proteins can be attached to an array by different means as will be apparent to a person skilled in the art. Proteins can for example be added to the array via a TAP-tag (as described in WO/0009716 and in Rigaut et al., 1999, Nature Biotechnol. 10:1030-1032) after the purification step or by another suitable purification scheme as will be apparent to a person skilled in the art.

Optionally, the proteins can be cross-linked to enhance their stability. Different methods to cross-link proteins are well known in the art. Reactive end-groups of cross-linking agents include but are not limited to -COOH, -SH, -NH2 or N-oxy-succinamate.

The spacer of the cross-linking agent should be chosen with respect to the size of the complex to be cross-linked. For small protein complexes, comprising only a few proteins, relatively short spacers are preferable in order to reduce the likelihood of cross-linking separate complexes in the reaction mixture. For larger protein complexes, additional use of larger spacers is preferable in order to facilitate cross-linking between proteins within the complex.

It is preferable to check the success-rate of cross-linking before linking the complex to the carrier.

As will be apparent to a person skilled in the art, the optimal rate of cross-linking need to be determined on a case by case basis. This can be achieved by methods well known in the art, some of which are exemplary described below.

A sufficient rate of cross-linking can be checked e.g. by analysing the cross-linked complex vs. a non-cross-linked complex on a denaturating protein gel.

If cross-linking has been performed successfully, the proteins of the complex are expected to be found in the same lane, whereas the proteins of the non-cross-linked complex are expected to be separated according to their individual characteristics. Optionally the presence of all proteins of the complex can be further checked by peptide-sequencing of proteins in the respective bands using methods well known in the art such as mass spectrometry and/or Edman degradation.

In addition, a rate of crosslinking which is too high should also be avoided. If cross-linking has been carried out too extensively, there will be an increasing amount of cross-linking of the individual protein complex, which potentially interferes with a screening for potential binding partners and/or modulators etc. using the arrays.

The presence of such structures can be determined by methods well known in the art and include e.g. gel-filtration experiments comparing the gel filtration profile solutions containing cross-linked complexes vs. uncross-linked complexes.

Optionally, functional assays as will be apparent to a person skilled in the art, some of which are exemplarily provided herein, can be performed to check the integrity of the complex.

Alternatively, the proteins or the protein can be expressed as a single fusion protein and coupled to the matrix as will be apparent to a person skilled in the art.

Optionally, the attachment of the proteins or antibody as outlined above can be further monitored by various methods apparent to a person skilled in the art. Those include, but are not limited to surface plasmon resonance (see e.g. McDonnel, 2001, Curr. Opin. Chem. Biol. 5:572-577; Lee, 2001, Trends Biotechnol. 19:217-222; Weinberger et al., 2000, 1:395-416; Pearson et al., 2000, Ann. Clin. Biochem. 37:119-145; Vely et al., 2000, Methods Mol. Biol. 121:313-321; Slepak, 2000, J. Mol Recognit. 13:20-26.

In a preferred embodiment of the present invention, in step a) the test compound is brought into contact with the Eph receptor and the interaction of the Eph receptor with the test compound is determined. This can be measured as described above.

Preferably, in step a) the ability of the Eph receptor modulator to modulate Eph receptor kinase activity is measured. Test for the measurement of protein kinase activity are known in the art. The test can e.g. be performed by the method described in Slon-Usakiewicz et al., 2004, J. Med. Chem. 47, 5094-5100.

In a preferred embodiment in step b) it is determined whether the Eph receptor modulator is capable of modulating, preferably lowering Abeta 42 production.

Exemplary assays useful for measuring the production of Abeta-40 and Abeta-42 peptides by ELISA include but are not limited to those described in Vassar R et al., 1999, Science, 286:735-41.

Exemplary assays useful for measuring the production of C-terminal APP fragments in cell lines or transgenic animals by western blot include but are not limited to those described in Yan R et al., 1999, Nature, 402:533-7.

Exemplary assays useful for measuring the proteolytic activity of beta- or gamma secretases towards bacterially expressed APP fragments in vitro include but are not limited to those described in Tian G et al., 2002, J Biol Chem, 277:31499-505.

To measure BACE1 activity, changes of the ratio between alpha- and beta-C-terminal APP fragments can be analyzed by Western Blotting (Blasko et al., J Neural Transm 111, 523); additional examples for BACE1 activity assays include but are not limited to: use of a cyclized enzyme donor peptide containing a BACE1 cleavage site to reconstitute and measure beta-galactosidase reporter activity (Naqvi et al., J Biomol Screen. 9, 398); use of quenched fluorimetric peptide substrates and fluorescence measurements (Andrau et al., J.Biol Chem 278, 25859); use of cell-based assays utilizing recombinant chimeric proteins, in which an enzyme (such as alkaline phosphatase) is linked via a stretch of amino acids, that contain the BACE1 recognition sequence, to a Golgi-resident protein (Oh et al., Anal Biochem, 323, 7); fluorescence resonance energy transfer (FRET)-based assays (Kennedy et al., Anal Biochen 319, 49); a cellular growth selection system in yeast (Luthi et al., Biochim Biophys Acta 1620,167).

Preferably, in the method of the invention, the Eph receptor is an EphB receptor. More preferred, the EphB receptor is selected from the consisting of EphB1, EphB2, EphB3, EphB4, EphB5, and EphB6.

EphB1 (aka ELK, NET, Hek6, EPHT2), one of the three most prominent Eph kinases in the brain (with A6, A8), recruits c-Src and p52Shc to activate MAPK/ERK and promotes chemotaxis (Vindis C, Cerretti DP, Daniel TO, Huynh-Do U (2003) EphB1 recruits c-Src and p52Shc to activate MAPK/ERK and promote chemotaxis. J Cell Biol. 162(4):661-71.). EphB1 (as is EphA4) is also expressed in platelets, where it has been hypothesized to contribute to play a supporting role in stable aggregation (Prevost N, Woulfe D, Tanaka T, Brass LF (2002) Interactions between Eph kinases and ephrins provide a mechanism to support platelet aggregation once cell-to-cell contact has occurred. Proc Natl Acad Sci U S A. 99(14):9219-24).

EphB2 (aka EPHT3; HEK5; Nuk; elk-related tyrosine kinase) is expressed in transcripts of 3 different sizes (4, 5, and 11 kb) in human brain and several other tissues, including heart, lung, kidney, placenta, pancreas, liver, and skeletal muscle, but the 11-kb DRT transcript is preferentially expressed in fetal brain. Steady-state levels of EphB2 mRNA in several tissues, including brain, heart, lung, and kidney, are greater in the midterm fetus than those in the adult (Ikegaki et al., 1995).

EphB2 has recently been implicated in the pathogenesis of a different types of tumors: For instance, it has been proposed that EphB2 signalling regulate migration and invasion of human glioma cells (Nakada et al., 2004); quantitative analysis of EphB2 and EphB4 expression is tumor specimen suggests that the two kinases be involved in the development of breast cancer and that both molecules could be potential predictive markers (Wu et al., 2004).

Besides roles during fetal development and in cancer, EphB2 function has recently been implicated in the formation of the astroglial- meningeal fibroblast scar (Bundesen et al., 2003). Inhibition of EphB2 kinase might therefore therapeutic opportunities in the prevention and/or therapy of spinal cord/CNS injury.

EphB4 (HTK, MYK1; TYRO11) is expressed in fetal, but not adult, brain, and in primitive and myeloid, but not lymphoid, hematopoietic cells as well as several malignant cell lines (Bennett et al., 1994). In some cases EphB4 has been found to be ectopically over-expressed in neoplastic conditions including colon cancer (Stephenson et al., 2001). In mice carrying a targeted deletion of EphB4 (and expressing LacZ instead) beta-galactosidase staining is confined to vascular endothelial cells in embryos and is preferentially found on veins. A targeted mutation in EphB4 essentially phenocopies the mutation in ephrin-B2, consistent with the idea that ephrin-B2-EphB4 mediate bidirectional signalling essential for angiogenesis (Gerety et al., 1999). Tumor growth has been shown to be dramatically reduced in soluble EphB4-expressing A375 tumors grown subcutaneously in nude mice compared to control tumors suggesting that anti-EphB4 strategies could be viable options in cancer therapy (Martiny-Baron et al., 2004). Interaction between ephrinB2 and EphB4 at the arterial-venous capillary interface, signaling through Ras/MAPK cascade in endothelial cells, is critical for proper embryonic capillary morphogenesis (Kim et al., 2002).

EphB6 lacks several invariant residues that have been shown to be essential for tyrosine kinase activity. Expression of its catalytic domain in mammalian cells resulted in no detectable tyrosine kinase activity (Matsuoka et al., 1997). Northern blot analysis of normal human adult tissues showed that EphB6 is expressed in all tissues examined, with very strong expression in the brain (135 kDa protein) and pancreas. Tang et al., 2000, found high levels of EphB6 expression in early stage neuroblastoma, but also suggested that high levels of EphB6 correlated with favourable outcome. Expression of exogenous EphB6 in SY5Y cells expressing little endogenous EphB6 resulted in inhibition of their clonogenicity in culture suggesting that EphB6 can suppress malignant phenotype of unfavorable neuroblastoma. To that same end, EphB6 expression is lost in metastatic melanoma (Hafner et al., 2003). Despite its lack of intrinsic kinase activity, (cross-linked) EphB6 does participate in signalling events: for instance, EphB6 cross-linking has been demonstrated to result in costimulation of T cells (Luo et al., 2002).

In a further preferred embodiment, the Eph receptor is an EphA receptor. More preferred, the EphA receptor is selected from the group consisting of EphA1, EphA2, EphA3, EphA4, EphA5, EphA6, EphA7, EphA8, EphA9, and EphA10.

EphA1 is expressed in liver, lung, kidney, and testes of rat; screening of 25 human cancers of various cell types has shown preferential expression in cells of epithelial origin (Maru et al., 1988). The EphA1 ligand ephrin-A2 forms a stable complex with the metalloprotease Kuzbanian (also known as ADAM10 - a form of APP gamma-secretase), involving interactions outside the cleavage region and the protease domain. Eph receptor binding has been demonstrated to trigger ephrin-A2 cleavage in a localized reaction specific to the cognate ligand - a proteolytic event important Eph's mediating axon withdrawal (Hattori et al., 2000).

EphA2 (aka Eck) is highly expressed in tissues that contain a high proportion of epithelial cells, including lung, skin, small intestine, and ovary (Lindberg and Hunter, 1990). As EphA2 is over-expressed in several types of tumors, this RTK is considered an attractive target for tumor therapy. To this end, soluble EphA2 and A3-receptors have been shown to inhibit tumor angiogenesis and progression in vivo (Brantley et al., 2002). Expression of EphA2 and estrogen receptor are inversely related in tumor tissue such as breast carcinoma and EphA2 over-expression has been shown to decrease tamoxifen sensitivity (Lu et al., 2003).

Any molecule known in the art can be tested for its ability to be a modulator or inhibitor according to the present invention. Candidate molecules can be directly provided to a cell expressing the Eph receptor, or, in the case of candidate proteins, can be provided by providing their encoding nucleic acids under conditions in which the nucleic acids are recombinantly expressed to produce the candidate protein.

In a preferred embodiment, the compound (and therefore also the Eph receptor modulator or inhibitor) is selected from the group consisting of antibodies, chemically modified ephrins, peptidomimetics of ephrins, antisense molecules, siRNA, binding peptides, and low molecular weight molecules (LMWs), preferably organic small molecule drugs.

In a preferred embodiment of the present invention, the Eph receptor modulator is an Eph receptor inhibitor.

According to the present invention the term "inhibitor" refers to a biochemical or chemical compound which preferably inhibits or reduces the activity of Eph. This can e.g. occur via suppression of the expression of the corresponding gene. The expression of the gene can be measured by RT-PCR or Western blot analysis. Furthermore, this can occur via inhibition of the activity, e.g. by binding to the Eph receptor.

Examples of such Eph receptor inhibitors are small preferably organic molecules, binding proteins or binding peptides directed against the Eph receptor, in particular against the active site of the Eph receptor, and nucleic acids directed against the Eph receptor gene.

The term "nucleic acids against the Eph receptor" refers to double-stranded or single stranded DNA or RNA, or a modification or derivative thereof which, for example, inhibit the expression of the Eph receptor gene or the activity of the Eph receptor and includes, without limitation, antisense nucleic acids, aptamers, siRNAs (small interfering RNAs) and ribozymes.

LMWs are molecules which are not proteins, peptides, antibodies or nucleic acids, and which exhibit a molecular weight of less than 5000 Da, preferably less than 2000 Da, more preferably less than 1000 Da, most preferably less than 500 Da. Such LMWs may be identified in High-Through-Put procedures starting from libraries. Such methods are known in the art and are discussed in detail below.

These nucleic acids can be directly administered to a cell, or which can be produced intracellularly by transcription of exogenous, introduced sequences.

An "antisense" nucleic acid as used herein refers to a nucleic acid capable of hybridizing to a sequence-specific portion of a component protein RNA (preferably mRNA) by virtue of some sequence complementarity. The antisense nucleic acid may be complementary to a coding and/or noncoding region of a component protein mRNA.

The antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides.

The nucleic acids, e.g. the antisense nucleic acids or siRNAs, can be synthesized chemically, e.g. in accordance with the phosphotriester method (see, for example, Uhlmann, E. & Peyman, A. (1990) Chemical Reviews, 90, 543-584). Aptamers are nucleic acids which bind with high affinity to a polypeptide, here the Eph receptor. Aptamers can be isolated by selection methods such as SELEX (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability.

Nucleic acids may be degraded by endonucleases or exonucleases, in particular by DNases and RNases which can be found in the cell. It is, therefore, advantageous to modify the nucleic acids in order to stabilize them against degradation, thereby ensuring that a high concentration of the nucleic acid is maintained in the cell over a long period of time (Beigelman et al. (1995) Nucleic Acids Res. 23:3989-94; WO 95/11910; WO 98/37240; WO 97/29116). Typically, such a stabilization can be obtained by introducing one or more internucleotide phosphorus groups or by introducing one or more non-phosphorus internucleotides.

Suitable modified internucleotides are compiled in Uhlmann and Peyman (1990), supra (see also Beigelman et al. (1995) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention. In general, the oligonucleotide can be modified at the base moiety, sugar moiety, or phosphate backbone.

The oligonucleotide may include other appending groups such as peptides, agents facilitating transport across the cell membrane (see, e.g., Letsinger et al., 1989, Proc. Natl. Acad. Sci. USA 86:6553-6556; Lemaitre et al., 1987, Proc. Natl. Acad. Sci. USA 84:648-652; International Patent Publication No. WO 88/09810) or blood-brain barrier (see, e.g., International Patent Publication No. WO 89/10134), hybridization-triggered cleavage agents (see, e.g., Krol et al., 1988, BioTechniques 6:958-976), or intercalating agents (see, e.g., Zon, 1988, Pharm. Res. 5:539-549).

In detail, the antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, D-mannosylqueosine, 5N-methoxycarboxymethyluracil, 5-methoxyuracil, 2-methyl-thio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

The use of suitable antisense nucleic acids is further described e.g. in Zheng and Kemeny (1995) Clin. Exp. Immunol., 100, 380-2; Nellen and Lichtenstein (1993) Trends Biochem. Sci., 18, 419-23, Stein (1992) Leukemia, 6, 697-74 or Yacyshyn, B. R. et al. (1998) Gastroenterology, 114, 1142).

In yet another embodiment, the oligonucleotide is a 2-a-anomeric oligonucleotide. An a-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, e.g., a peptide, hybridization-triggered cross-linking agent, transport agent, hybridization-triggered cleavage agent, etc.

Throughout the invention, oligonucleotides of the invention may be synthesized by standard methods known in the art, e.g., by use of an automated DNA synthesizer (such as are commercially avail-able from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. USA 85:7448-7451), etc.

In a specific embodiment, the antisense oligonucleotides comprise catalytic RNAs, or ribozymes (see, e.g., International Patent Publication No. WO 90/11364; Sarver et al., 1990, Science 247:1222-1225). In another embodiment, the oligonucleotide is a 2'-0-methylribonucleotide (Inoue et al., 1987, Nucl. Acids Res. 15:6131-6148), or a chimeric RNA-DNA analog (Inoue et al., 1987, FEBS Lett. 215:327-330).

In an alternative embodiment, the antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the component protein. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells. Expression of the sequences encoding the antisense RNAs can be by any promoter known in the art to act in mammalian, preferably human, cells. Such promoters can be inducible or constitutive. Such promoters include, but are not limited to, the SV40 early promoter region (Bemoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. USA 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a component protein gene, preferably a human gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a component protein RNA it may contain and still form a stable duplex (or triplex, as the case may be). One skilled in the art can ascertain a tolerable degree of mismatch by use of standard procedures to determine the melting point of the hybridized complex.

The production and use of siRNAs as tools for RNA interference in the process to down regulate or to switch off gene expression, here Eph receptor gene expression, is e.g. described in Elbashir, S. M. et al. (2001) Genes Dev., 15, 188 or Elbashir, S. M. et al. (2001) Nature, 411, 494. Preferably, siRNAs exhibit a length of less than 30 nucleotides, wherein the identity stretch of the sense strang of the siRNA is preferably at least 19 nucleotides.

Ribozymes are also suitable tools to inhibit the translation of nucleic acids, here the Eph receptor gene, because they are able to specifically bind and cut the mRNAs. They are e.g. described in Amarzguioui et al. (1998) Cell. Mol. Life Sci., 54, 1175-202; Vaish et al. (1998) Nucleic Acids Res., 26, 5237-42; Persidis (1997) Nat. Biotechnol., 15, 921-2 or Couture and Stinchcomb (1996) Trends Genet., 12, 510-5.

Pharmaceutical compositions, comprising an effective amount of a nucleic acid in a pharmaceutically acceptable carrier, can be administered to a patient having a disease or disorder that is of a type that expresses or overexpresses a protein as described in the present invention.

The amount of the nucleic acid that will be effective in the treatment of a particular disorder or condition will depend on the nature of the disorder or condition, and can be determined by standard clinical techniques. Where possible, it is desirable to determine the nucleic acid cytotoxicity in vitro, and then in useful animal model systems, prior to testing and use in humans.

Pharmaceutical compositions comprising nucleic acids are administered via liposomes, microparticles, or microcapsules. In various embodiments, it may be useful to use such compositions to achieve sustained release of the nucleic acids. In a specific embodiment, it may be desirable to utilize liposomes targeted via antibodies to specific identifiable central nervous system cell types (Leonetti et al., 1990, Proc. Natal. Acad. Sci. U.S.A. 87:2448-2451; Renneisen et al., 1990, J. Biol. Chem. 265:16337-16342).

The term "binding protein" or "binding peptide" refers to a class of proteins or peptides which bind and inhibit the Eph receptor, and includes, without limitation, polyclonal or monoclonal antibodies, antibody fragments and protein scaffolds directed against the Eph receptor.

According to the present invention the term antibody or antibody fragment is also understood as meaning antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884), preferably produced with the help of a FAB expression library.

As an alternative to the classical antibodies it is also possible, for example, to use protein scaffolds against the Eph receptors, e.g. anticalins which are based on lipocalin (Beste et al. (1999) Proc. Natl. Acad. Sci. USA, 96, 1898-1903). The natural ligand-binding sites of the lipocalins, for example the retinol-binding protein or the bilin-binding protein, can be altered, for example by means of a "combinatorial protein design" approach, in such a way that they bind to selected haptens, here to the Eph receptor (Skerra, 2000, Biochim. Biophys. Acta, 1482, 337-50). Other known protein scaffolds are known as being alternatives to antibodies for molecular recognition (Skerra (2000) J. Mol. Recognit., 13, 167-187).

The procedure for preparing an antibody or antibody fragment is effected in accordance with methods which are well known to the skilled person, e.g. by immunizing a mammal, for example a rabbit, with the Eph receptor, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromato-graphy. Monoclonal antibodies can, for example, be prepared in accordance with the known method of Winter & Milstein (Winter, G. & Milstein, C. (1991) Nature, 349, 293-299).

In detail, polyclonal antibodies can be prepared as described above by immunizing a suitable subject with a polypeptide as an immunogen. Preferred polyclonal antibody compositions are ones that have been selected for antibodies directed against a polypeptide or polypeptides of the invention. Particularly preferred polyclonal antibody preparations are ones that contain only antibodies directed against a given polypeptide or polypeptides. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes, thereby generating a substantially purified antibody composition, i.e., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein, 1975, Nature 256:495-497, the human B cell hybridoma technique (Kozbor et al., 1983, Immunol. Today 4:72), the EBV-hybridoma technique (Cole et al., 1985, Monoclonal Antibodies and Cancer Therapy, Alan R. Liss, Inc., pp. 77-96) or trioma techniques. The technology for producing hybridomas is well known (see generally Current Protocols in Immunology 1994, Coligan et al. (eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, Bio/Technology 9:1370-1372; Hay et al., 1992, Hum. Antibod. Hybridomas 3:81-85; Huse et al., 1989, Science 246:1275-1281; Griffiths et al., 1993, EMBO J. 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, e.g., Cabilly et al., U.S. Patent No. 4,816,567; and Boss et al., U.S. Patent No. 4,816,397 ) Humanized antibodies are antibody molecules from non-human species having one or more complementarily determining regions (CDRs) from the non-human species and a framework region from a human immunoglobulin molecule. (See, e.g., Queen, U.S. Patent No. 5,585,089) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559); Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar, 1995, Int. Rev. Immunol. 13:65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope. (Jespers et al., 1994, Bio/technology 12:899-903).

Antibody fragments that contain the idiotypes of the protein can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments.

In the production of antibodies, screening for the desired antibody can be accomplished by techniques known in the art, e.g., ELISA (enzyme-linked immunosorbent assay). To select antibodies specific to a particular domain of the protein, or a derivative thereof, one may assay generated hybridomas for a product that binds to the fragment of the protein, or a derivative thereof, that contains such a domain. For selection of an antibody that specifically binds a protein as described in the present invention, or a derivative, or homologue thereof, but which does not specifically bind to the individual proteins of the protein, or a derivative, or homologue thereof, one can select on the basis of positive binding to the proten and a lack of binding to the individual protein components.

The foregoing antibodies can be used in methods known in the art relating to the localization and/or quantification of the given protein or proteins, e.g., for imaging these proteins, measuring levels thereof in appropriate physiological samples (by immunoassay), in diagnostic methods, etc. This hold true also for a derivative, or homologue thereof of a protein.

The method of the invention is well suited to screen chemical libraries for molecules which modulate, e.g., inhibit, antagonize, or agonize, the amount of or the activity of a protein. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention: Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et al., 1994, J. Med. Chem. 37:1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, - amino isobutyric acid, 4-aminobutyric acid, Abu, 2-amino butyric acid; -Abu, -Ahx, 6-amino hexanoic acid; Aib, 2-amino isobutyric acid; 3-amino propionic acid; ornithine; norleucine; norvaline, hydroxyproline, sarcosine, citrulline, cysteic acid, t-butylglycine, t-butylalanine, phenylglycine, cyclohexylalanine, β-alanine, designer amino acids such as β-methyl amino acids, C-methyl amino acids, N-methyl amino acids, fluoro-amino acids and amino acid analogs in general. Furthermore, the amino acid can be D (dextrorotary) or L (levorotary).

In a specific embodiment, fragments and/or analogs of proteins of the invention, especially peptidomimetics, are screened for activity as competitive or non-competitive inhibitors of complex activity or formation. A method for the identification of Ephrin mimetic peptides is described in Koolpe et al., 2002, J. Biol. Chem. 277, No. 49, 46974-46979. It has been envisioned that such peptides could be used to selectively deliver agents to Eph receptor-expressing tissues and modify Eph signaling in therapies for cancer, pathological angiogenesis, and nerve regeneration.

In another embodiment of the present invention, combinatorial chemistry can be used to identify modulators of a protein. Combinatorial chemistry is capable of creating libraries containing hundreds of thousands of compounds, many of which may be structurally similar. While high throughput screening programs are capable of screening these vast libraries for affinity for known targets, new approaches have been developed that achieve libraries of smaller dimension but which provide maximum chemical diversity. (See e.g., Matter, 1997, J. Med. Chem. 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest. The fingerprints are compared with fingerprints obtained from other compounds known to react with the protein of interest to predict whether the library compound might similarly react. For example, rather than testing every ligand in a large library for interaction with a protein component, only those ligands having a fingerprint similar to other compounds known to have that activity could be tested. (See, e.g., Kauvar et al., 1995, Chem. Biol. 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, Kurtz, Stanker and Skerritt (eds), 1995, AOAC: Wshington, D.C., 305-312).

Kay et al. (1993, Gene 128:59-65) disclosed a method of constructing peptide libraries that encode peptides of totally random sequence that are longer than those of any prior conventional libraries. The libraries disclosed in Kay et al. encode totally synthetic random peptides of greater than about 20 amino acids in length. Such libraries can be advantageously screened to identify protein modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

It is disclosed a method for preparing a pharmaceutical composition for the treatment of neurodegenerative diseases, preferably Alzheimer's disease, comprising the following steps:
a) identifying an Abeta peptide lowering agent / gamma secretase and/or beta secretase modulator, preferably inhibitor according to the invention as described above, and
b) formulating the gamma secretase and/or beta secretase modulator to a pharmaceutical composition.

This method further comprises the step of mixing the identified molecule with a pharmaceutically acceptable carrier.

Therefore, there are described pharmaceutical compositions, which may be administered to a subject in an effective amount. In a preferred aspect, the therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the therapeutic can be delivered via a controlled release system. In one embodiment, a pump may be used (Langer, supra; SeRon, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a specific embodiment where the therapeutic is a nucleic acid, preferably encoding a protein therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid therapeutic can be introduced intracellularly and incorporated by homologous recombination within host cell DNA for expression.

In general, the pharmaceutical compositions comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil . Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol. The composition, if desired, can also contain minor amounts of wetting or emulsifying agents, or pH buffering agents. These compositions can take the form of solutions, suspensions, emulsions, tablets, pills, capsules, powders, sustained-release formulations . The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

It is also provided a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits can also contain expression vectors encoding the protein(s). Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

It is disclosed the use of an Eph receptor inhibitor, wherein the Eph receptor inhibitor is also a gamma secretase and/or a beta secretase inhibitor, for the preparation of a medicament for the therapy of a neurodegenerative disease, preferably Alzheimer's disease, wherein the inhibitor is an intracellular inhibitor and not a compound comprising the following pharmacophore: 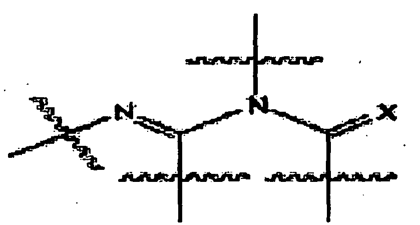 wherein X is CH-, O, NH or N-CO-

This pharmacophore has been described in US2004/0028673. The passage below are taken from this application and indicate those compounds which are preferably excluded from the present invention.

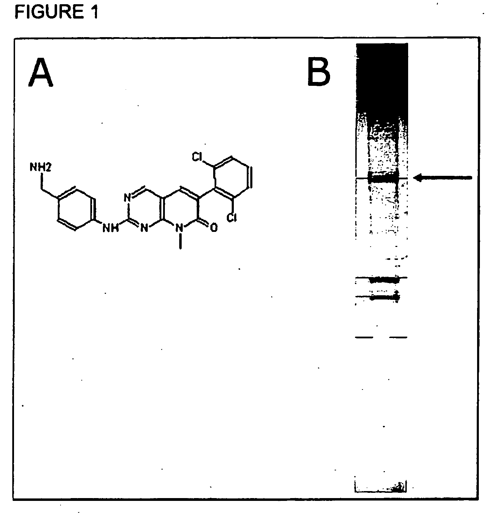

Preferably, the inhibitor is not a pharmacophore containing compound which comprises a compound of Figure 1 or a pharmaceutically acceptable salt thereof : 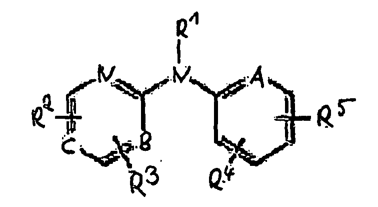 A is CH or N;B and C are independently CH, N or N+-O-; R'is H, SO2Ra, (C=O) rOsRa ; R2, R3, R'and R'are independently H, OH, CHO, CN, halogen, (C=O) rOs (C1- C10) alkyl, (C=O) rOs (C,-C, o) alkenyl, (C=O) rOs (C2-C10) alkynyl, (C=O) 0, cyclolkyl, (C=O) rOscycloalkenyl, (C=O)rOscycloalkynyl, (C=O)rOsheterocycyl, (C=O)rOsaryl, (C=O) rOsheteroaryl, (C=O)rOsperfluoroalkyl or (C0-C6)alkyl-NRbRc, wherein said alkykl, alkenyl, alkynyl, cycyloalkyl, cycloalkenyl, cycloalkynyl, heterocycyl, aryl, heteroaryl and perfluoroalkyl is optionally substituted with one or more substituents selected from R6 ;R6 is (C=O)rOsNRaRb, (C=O)rOsaryl, (C=O)rOsheterocycyl, halogen, OH, oxo, (C=O) rOs (C,-C3) perfluoroalkyl, (C=O) O, (C-C6) alkyl, CHO, CO2H, CN, (C0-C6)alkyl- NRbRc or (C1-C6)alkyl-heterocycyl, wherein said alkyl-heterocycyl is optionally substituted with OH; Ra is (C1-C6)alkyl, aryl or heterocycyl ; and Rb and Rc independently are H, (C=O) rOs (C1-C10) alkyl, SO2Ra, (C=O)rOsheterocycyl, (C=O) rOsaryl, (C=O)rOsheteroaryl or CO2Ra, wherein r and s independently are 0 or 1 and said alkyl, heterocycyl, aryl or heteroaryl is optionally substituted with one or more substituents selected from R6.

In another embodiment, A is CH, B is N and C is CH.In another embodiment, R'is H.

In another embodiment, R is H and R3 is heteroaryl.

In another embodiment, R4 is (C=O)rOs(C1-C10) alkyl and RIS is (C0-C6)alkyl-NRbRc.

In another embodiment, R'is 3-pyridinyl.

In another embodiment, r is 0, s is 0 and (C,-Clo) alkyl is methyl.

In another embodiment, (C0-C6)alkyl is a direct bond (C0), Rb is H and Kc is (C=O) 0, heteroaryl or (C=O) rOsheterocycyl.

In another embodiment, (C=O) rOsheterocycyl is 4-hydioxy-1-piperazino, as illustrated below or a pharmaceutically acceptable salt thereof.

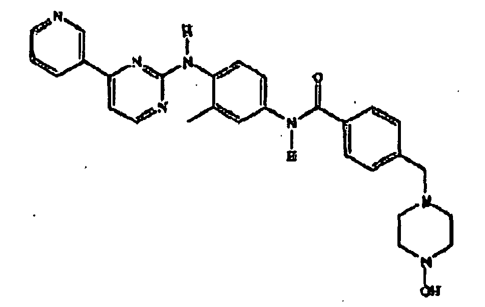

In another embodiment, (C=O) rOsheteroaryl is 3-pyrindinyl, as illustrated below or a pharmaceutically acceptable salt thereof.

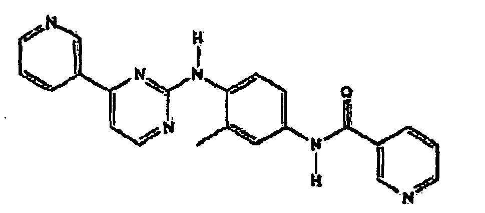

In a further preferred embodiment, the inhibitor is not a pharmacophore containing compound which comprises a compound of Figure II or a pharmaceutically acceptable salt thereof :

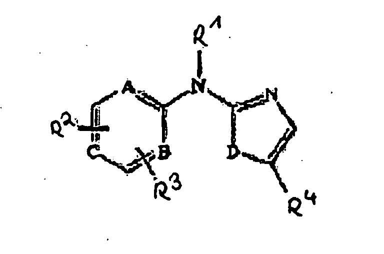

A, B and C are independently CH, N or N+-O-; D is O, S or N-R5 ; R'is H, SO2Ra, (C=O) W or CO2Ra ; R2, R3 and R4 are independently H, OH, CHO, CN, halogen, (C=O) rOs (C1-C10) alkyl, (C=O) rOs (C2-C10)alkenyl, (C=O)rOs(C2-C10)alkynyl, (C=O)rOscycloalkyl, (C=O) rOscycloalkenyl, (C=O)rOscycloalkynyl, (C=O)rOsheterocycyl, (C=O)rOsaryl, (C=O) 0, heteroaryl, (C-0), 0, perfluoroalkyl or (C0-C6)alkyl-NRbRc, wherein said alkykl, alkenyl, alkynyl, cycyloalkyl, cycloalkenyl, cycloalkynyl, heterocycyl, aryl, heteroaryl and perfluoroalkyl is optionally substituted with one or more substituents selected from R6 ; R'is H, aryl or (C1-C6)alkyl ; R6 is (C=O) rOsNRaRb, (C=O)rOsaryl, (C=O)rOsheterocycyl, halogen, OH, oxo, (C=O) rOs (C,-C3) perfluoroalkyl, (C=O) rOs (C1-C6) alkyl, CHO, CO2H, CN, (C0-C6) alkyl- NRbRc or (C1-C6)alkyl-heterocycyl ; Ra is (C1-C6)alkyl, aryl or heterocycyl ; and Rb and Rc independently are H, (C=O) rOs (C1-C10) alkyl, SO2Ra, (C=O) rOsheterocycyl, (C=O) rOsaryl, (C=O)rOsheteroaryl or CO2Ra, wherein r and s independently are 0 or 1 and said alkyl, heterocycyl, aryl or heteroaryl is optionally substituted with one or more substituents selected from R6.

In another embodiment, R'is H.

In another embodiment, Ruz ils H and R3 is (Co-C6) alkyl-NRbR.

In another embodiment, R4 is heteroaryl.

In another embodiment, said pharmacophore containing compound, comprises a compound of Figure III or a pharmaceutically acceptable salt thereof

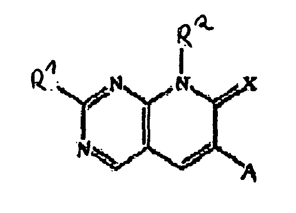

A is aryl or heteroaryl, wherein said aryl or heteroaryl is optionally substituted with one or more substituents selected from R3 ; X is NH, N-acyl, O or S; R1 and R2 are independently H, OH, CHO, CN, halogen, (C=O) rOs (C,-C, o) alkyl, (C=O) rOs (C2-C10) alkenyl, (C=O) O, (C2-C10)alkynyl, (C=O)rOscycloalkyl, (C=O) rOscycloalkenyl, (C=O)rOscycloalkynyl, (C=O)rOsheterocycyl, (C=O)rOsaryl, (C=O) rOsheteroaryl, (C=O)rOsperfluoroalkyl or (C0-C6)alkyl-NRaRb, wherein said alkykl, alkenyl, alkynyl, cycyloalkyl, cycloalkenyl, cycloalkynyl, heterocycyl, aryl, heteroaryl and perfluoroalkyl is optionally substituted with one or more substituents selected from R' ; R3 is (C=O) rOsNRaRb, (C=O)rOsaryl, (C=O)rOsheterocycyl, halogen, OH, oxo, (C=O) rOs (C1-C3) perfluoroalkyl, (C=O) rOsSt(C1-C6)alkyl, CHO, CO2H, CN, (Co-C6) alkyl- NRaRb or (C1-C6)alkyl-heterocycyl ; Ra and Rb independently are H, (C=O) rOs (C1-C10)alkyl, SO2R1, (C=O)rOsheterocycyl, (C=O) rOsaryl, (C=O)rOsheteroaryl or CO2R', wherein r, s and t independently are 0 or 1 and said alkyl, heterocycyl, aryl or heteroaryl is optionally substituted with one or more substituents selected from R3.

In another embodiment, A is aryl.

In another embodiment, the aryl is 2,5-dichlorophenyl.

In another embodiment, R'is (Co-C6) alkyl-NRaRb and R2 is (C=O) O, (C1-C10)alkyl.

In another embodiment, (C=O)rOs(C1-C10)alkyl is methyl.

In another embodiment, (C0-C6)alkyl-NRaRb is 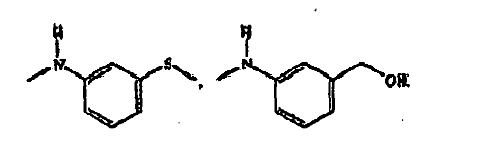 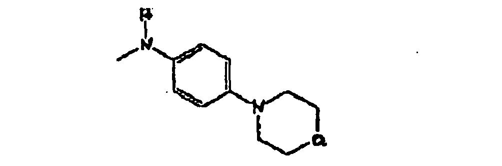 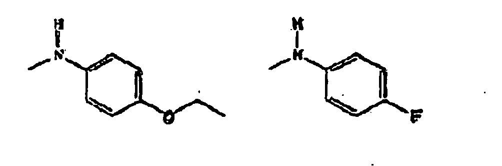 or a pharmaceutically acceptable salt thereof.

Preferably, the inhibitor is not imatinib mesylate.

These above compounds are preferably explicitly disclaimed from the present invention.

In a preferred embodiment of the use, the Eph receptor is an EphB receptor. More preferred, the EphB receptor is selected from the group consisting of EphB1, EphB2, EphB3, EphB4, EphB5, and EphB6.

In another preferred embodiment of the use, the receptor is an EphA receptor. More preferred, the EphA receptor is selected from the group consisting of EphA1, EphA2, EphA3, EphA4, EphA5, EphA6, Eph7, EphA8, EphA9, and EphA10.

With respect to the inhibitor, all embodiments as described above for the methods of the invention also apply to this use. As indicated above, the inhibitor is preferably selected from the group consisting of antibodies, antisense oligonucleotides, siRNA, low molecular weight molecules (LMWs), binding peptides, aptamers, ribozymes and peptidomimetics.

Preferably, the inhibitor is a protein kinase inhibitor as discussed above. Even more preferred, the inhibitor has the structure 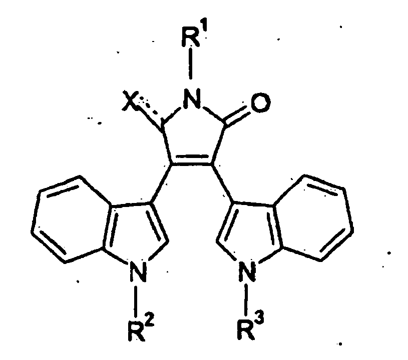 wherein
X stands for 2 H-atoms; 1 H-atom and 1 OH-group; or O; and the dotted line stands for a double bond which is optionally present;
R¹ is H, or a linear or branched C₁-C₄ alkyl group which is preferably unsubstituted; or a C₂-C₄ alkenyl group which is preferably unsubstituted;
R², R³ are independently of each other selected from H; linear or branched, cyclic or noncyclic C₁-C₆ alkyl groups; or C₂-C₆ alkenyl groups; which alkyl or alkenyl groups may be substituted by: an amino group of the formula NR⁴R⁵; a 5- or 6-membered N-heterocycle; SR⁶; or a 5- or 6-membered S-heterocycle; and wherein at least one of R² and R³ preferably is H;
R⁴, R⁵ are independently of each other selected from H; C₁-C₄ alkyl; and C₂-C₄ alkenyl;
R⁶ being selected from H; C₁-C₄ alkyl; or -C(NH)(NH₂) or a derivative thereof wherein at least one of the H-atoms is substituted by C₁-C₄ alkyl or C₂-C₄ alkenyl.

It is described the use of an Eph receptor inhibitor, wherein the Eph receptor inhibitor is also a gamma secretase and/or a beta secretase inhibitor, for the preparation of a medicament for the therapy of a neurodegenerative disease, preferably Alzheimer's disease, wherein the inhibitor is an extracellular inhibitor.

Also with respect to the inhibitor, all embodiments as described above for the methods of the invention also apply to this use. Preferably, this inhibitor is selected from the group consisting of antibodies, chemically modified ephrins, peptidomimetics of ephrins, binding peptides, and low molecular weight molecules (LMWs), preferably organic small molecule drugs.

Preferably, the inhibitor blocks the interaction of the Eph receptor with its natural ligand.

It is also disclosed a pharmaceutical composition comprising an Eph receptor inhibitor as defined in the context of the uses of the present invention, and a pharmaceutical composition obtainable by the method for the preparation of a pharmaceutical composition of the invention.

With respect to the pharmaceutical composition, all embodiments as disclosed above for the method for the preparation of a pharmaceutical composition also apply.

It is disclosed a pharmaceutical composition for the treatment of a neurodegenerative disease such as Alzheimer's disease and related neurodegenerative disorders.

It is further referred to a method for treating or preventing a neurodegenerative disease, preferably Alzheimer's disease comprising administering to a subject in need of such treatment or prevention a therapeutically effective amount of a pharmaceutical composition. With respect to this method of the invention, all embodiments described above for the uses, methods or pharmaceutical compositions also apply.

It is described the use of an Eph receptor modulator for the modulation of beta secretase and/or gamma secretase activity in vitro. For example, it is encompassed within the present invention to modulate, preferably inhibit beta secretase and/or gamma secretase activity in cell cultures by the Eph interacting molecule. All embodiments with respect to the Eph interacting molecule as described above also apply to this use of the invention.

### Figures:

### Figure 1:

Chemical proteomics with immobilized amine-containing analog of PD173955 identifies Eph kinases.
A, a primary amine-containing analog of PD 173955 synthesized and immobilized on sepharose beads;
B, beads were subsequently incubated with 50 mg mouse brain lysate; bound proteins were separated by SDS-PAGE; bands were cut out and proteins were identified by LC-MS/MS. Eph kinases were identified in the band marked by an arrow.

### Figure 2:

Gleevec and PD179355 inhibit cellular tyrosine phosphorylation of Eph kinase.

EphB2-TAP (A, B) and EphA4-TAP (C,D) stably expressed in SK-N-BE2 neuroblastoma cells are phosphorylated on tyrosine residues under resting conditions. Treatment of cells with Gleevac (A, C) or PD179355 (B, D) for 10 min. at 37 oC inhibits tyrosine phosphorylation of Eph kinases in a concentration-dependent manner. Following treatment, cells were lysed in a detergent-containing buffer and TAP-tagged Eph kinases were captured with IgG beads. Bound TAP-tagged proteins were analyzed by SDS-PAGE and Western blotting, utilizing either anti-phosphotyrosine or anti-TAP antibodies for detection.

### Example

The following examples refer to all embodiments of the invention and especially to the embodiments as claimed in the claims.

### Example 1:

Identification of specific compound-binding proteins by chemical proteomics.

To identify novel kinase targets of PD173955 that might be involved in its Abeta-lowering effect, a chemical proteomics procedure is employed (see below). PD173955-binding proteins are subsequently separated by SDS-PAGE, suitable gel bands cut out and analyzed by LC-MS/MS.

Result: Use of a chemical proteomics discovery platform with an immobilized PD179355 analog as a screening tool (Figure 1) identifies several Eph kinases (as summarized in Table I) as compound-specific and high-affinity (data not shown) binding proteins.

### 1 Immobilization of small molecule amine compounds

NHS-activated Sepharose 4 Fast Flow ( Amersham Biosciences, 17-0906-01) is equilibrated with anhydrous DMSO (Dimethylsulfoxid, Fluka, 41648, H₂0 <= 0.005%). 1 ml of settled beads are placed in 15 ml Falcon tube, compound stock solution (usually 100 mM in DMF or DMSO) is added (final conc. 0.2-2 µmol/ml beads) as well as 15 µl of triethylamine (SIGMA, T-0886, 99% pure). Beads are incubated at RT in darkness on an end-over-end shaker (Roto Shake Genie, Scientific Industries Inc.) for 16 - 20 h. Coupling efficiency is determined by HPLC. Non-reacted NHS-groups are blocked by incubation with aminoethanol at RT on the end-over-end shaker over night. Washed beads are stored in isopropanol.

### 2 Lysis of mouse brain in 1 % dodecyl-maltoside (DDM-) containing buffer

Mouse brains are homogenized in a tissue grinder in lysis buffer (1 per 5 ml): 50 mM Tris-HCl, 1% DDM, 5% glycerol, 150 mM NaCl, 1.5 mM MgCl2, 25 mM NaF, 1 mM sodium vanadate, I mM DTT, pH 7.5 + 1 complete EDTA-free tablet (protease inhibitor cocktail, Roche Diagnostics, 1 873 580) / 25 ml buffer. Material is dounced 10x using a mechanized POTTER S, transferred to 50 ml falcon tubes; incubated for 30 min on ice and spun down for 10 min at 20,000 g at 4°C (10,000 rpm in Sorvall SLA600, precooled). Supernatant is transfered to a UZ-polycarbonate tube (Beckmann, 355654) and spun for 1 h at 100.000 g at 4°C (33.500 rpm in Ti50.2, precooled). Supernatant is again transferred to a fresh 50 ml falcon tube, protein concentration is determined by Bradford assay (BioRad) and 50 mg aliquots are prepared.

### 3 Compound pull-down experiment

NHS-beads with immobilized compound are equilibrated in lysis buffer and incubated with 50 mg mouse brain lysate on an end-over-end shaker (Roto Shake Genie, Scientific Industries Inc.) for 2h at 4°C. Beads are collected, transfered to Mobi-columns (mobiol columns + filter 90 um, MoBiTech 10055) and washed with 5 ml lysis buffer containing 0.4% detergent, followed by 5 ml (followed by 2.5 ml) lysis buffer with 0.2 % detergent. To elute bound protein, 60 ul 2 x sample buffer is added, the column is heated for 30 min at 50°C and the eluate transferred to a microfuge tube by centrifugation.

### 4 Protein Identification by Mass Spectrometry

### 4.1 Protein digestion prior to mass spectrometric analysis

Gel-separated proteins are reduced, alkylated and digested in-gel, essentially following the procedure described by Shevchenko et al., 1996, Anal. Chem. 68:850-858. Briefly, gel-separated proteins are excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 min). Reduced and alkylated proteins are digested in-gel with porcine trypsin (12.5 ng/µl; Promega) in 5mM ammonium bicarbonate. Digestion is allowed to proceed for 4 hours at 37°C and the reaction is then stopped using 5 µl 5% formic acid.

### 4.2 Sample preparation prior to analysis by mass spectrometry

Gel plugs are extracted twice with 20 µl 1% TFA and pooled with acidified digest supernatants. Samples are dried in a vaccum centrifuge and resuspended in 13 µl 1% TFA.

### 4.3. Mass spectrometric data acquisition

Peptide samples are injected into a nano LC system (CapLC, Waters or Ultimate, Dionex) which is directly coupled either to a quadrupole TOF (QTOF2, QTOF Ultima, QTOF Micro, Micromass or QSTAR Pulsar, Sciex) or ion trap (LCQ Deca XP) mass spectrometer. Peptides are separated on the LC system using a gradient of aqueous and organic solvents (see below). Solvent A is 5% acetonitrile in 0.5% formic acid and solvent B is 70% acetonitrile in 0.5% formic acid.

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

Peptides eluting off the LC system are partially sequenced within the mass spectrometer.

### 4.4. Protein identification

The peptide mass and fragmentation data generated in the LC-MS/MS experiments are used to query FASTA-formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins are identified by correlating the measured peptide mass and fragmentation data with the same data computed from the entries in the database using the software tool Mascot (Matrix Science; Perkins et al., 1999, Electrophoresis 20:3551-3567). Search criteria vary depending on which mass spectrometer is used for the analysis.

### Example 2:

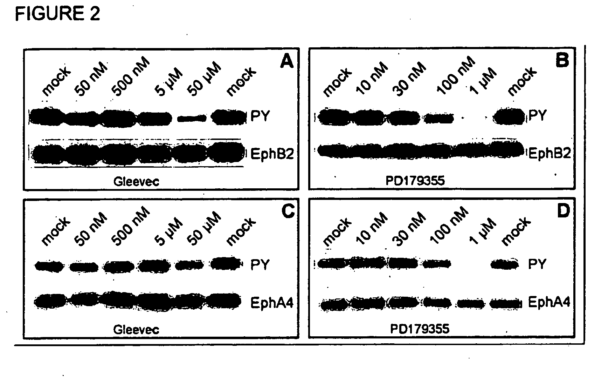

Characterization of inhibitory potency of compounds against Eph kinases
Result: PD179355 and Gleevac are potent inhibitors of autophosphorylation of TAP-tagged Eph kinases in cells (Figure 2) and in-vitro (data not shown)
Part I: Generation of cell lines stably expressing TAP-tagged proteins
To generate cell lines stably expressing TAP-tagged proteins for use in cellular assays, some aspects of TAP-technology, which is more fully described in EP 1 105 508 B1 and in Rigaut, et al., 1999, Nature Biotechnol. 17:1030-1032, respectively, are used:

### 1 Construction of TAP-tagged protein

The cDNAs encoding the complete ORF are obtained by RT-PCR. Total RNA is prepared from appropriate cell lines using the RNeasy Mini Kit (Qiagen). Both cDNA synthesis and PCR are performed with the SUPERSCRIPT One-Step RT-PCR for Long templates Kit (Life Technologies) using gene-specific primers. After 35-40 cycles of amplification PCR-products with the expected size are gel-purified with the MinElute PCR Purification Kit (Qiagen) and, if necessary, used for further amplification. Low-abundant RNAs are amplified by nested PCR before gel-purification. Restriction sites for NotI are attached to PCR primers to allow subcloning of amplified cDNAs into the retroviral vectors pIE94-N/C-TAP thereby generating N-terminal fusions with the TAP-tag (Rigaut et al., 1999, Nature Biotechnol. 17:1030-1032).
Clones are analyzed by restriction digest, DNA sequencing and by in vitro translation using the TNT T7 Quick Coupled Transcription/Translation System (Promega inc.). The presence of the proteins is proven by Western blotting using the protein A part of the TAP-tag for detection. Briefly, separation of proteins by standard SDS-PAGE is followed by semi-dry transfer onto a nitrocellulose membrane (PROTRAN, Schleicher&Schuell) using the MultiphorII blotting apparatus from Pharmacia Biotech. The transfer buffer is: 48 mM Tris, 39 mM glycine, 10% methanol and 0,0375% sodium dodecylsulfate. After blocking in phosphate-buffered saline (PBS) supplemented with 10% dry milk powder and 0,1% Tween 20 transferred proteins are probed with the Peroxidase-Anti-Peroxidase Soluble Complex (Sigma) diluted in blocking solution. After intensive washing immunoreactive proteins are visualized by enhanced chemiluminescence (ECL; Amersham Pharmacia Biotech).

### 2 Preparation of Virus and infection

As a vector, a MoMLV-based recombinant virus is used.

The preparation is carried out as follows:

### 2.1. Preparation of Virus

293 gp cells are grown to 100% confluency. They are split 1:5 onto poly-L-Lysine plates (1:5 diluted poly-L-Lysine [0.01% stock solution, Sigma P-4832] in PBS, left on plates for at least 10 min.). On Day 2, 63 microgram of retroviral Vector DNA together with 13 microgram of DNA of plasmid encoding an appropriate envelope protein is transfected into 293 gp cells (Somia, et al., 1999, Proc. Natl. Acad. Sci. USA 96:12667-12672; Somia, et al. 2000, J. Virol. 74:4420-4424). On Day 3, the medium is replaced with 15 ml DMEM + 10% FBS per 15-cm dish. On Day 4, the medium containing viruses (supernatant) is harvested (at 24 h following medium change after transfection). In case a second collection is planned, DMEM 10 % FBS is added to the plates and the plates were incubated for another 24 h. All collections are done as follows: The supernatant is filtered through 0.45 micrometer filter (Coming GmbH, cellulose acetate, 431155). The filter is placed into conical polyallomer centrifuge tubes (Beckman, 358126) that are placed in buckets of a SW 28 rotor (Beckman). The filtered supernatant is centrifuged at 19400 rpm in the SW 28 rotor, for 2 hours at 21 degree Celsius. The supernatant is discarded. The pellet containing viruses is resuspended in a small volume (for example 300 microliter) of Hank's Balanced Salt Solution [Gibco BRL, 14025-092], by pipetting up and down 100-times, using an aerosol-safe tip. The viruses are used for transduction as described below.

### 2.2. Infection

Cells are plated one day prior to infection onto one well of a 6-well plate. 4 hours before infection, the old medium on the cells is replaced with fresh medium. Only a minimal volume is added, so that the cells are completely covered (e.g. 700 µl). During infection, the cells are actively dividing.

### A description of the cells and their growth conditions is given further below ("3. Cell lines")

To the concentrated virus, polybrene (Hexadimethrine Bromide; Sigma, H 9268) is added to achieve a final concentration of 8 microgram/ml (this is equivalent to 2.4 microliter of the 1 milligram/ml polybrene stock per 300 microliter of concentrated retrovirus). The virus is incubated in polybrene at room temperature for 1 hour. For infection, the virus/polybrene mixture is added to the cells and incubated at 37 degree Celsius at the appropriate CO2 concentration for several hours (e.g. over-day or overnight). Following infection, the medium on the infected cells is replaced with fresh medium. The cells are passaged as usual after they become confluent. The cells contain the retrovirus integrated into their chromosomes and stably express the gene of interest.

### 2.3. Cell lines

For expression, SKN-BE2 cells are used. SKN-BE2 cells (American Type Culture Collection-No. CRL-2271) are grown in 95% OptiMEM + 5% iron-supplemented calf serum.

The expression pattern of the TAP-tagged proteins is checked by immunoblot-analysis as described in Example 2, Nr. 3.3. and/or by immunofluorescence as described in Example 2, Nr. 3.1 or 3.2.

### 3 Checking of expression pattern of TAP-tagged proteins

The expression pattern of the TAP-tagged protein is checked by immunoblot analysis and/or by immunofluorescence. Immunofluorescence analysis is either carried out according to No. 1 or to No. 2, depending on the type of the TAP-tagged protein. Immunoblot analysis is carried out according to No. 3.

### 3.1 Protocol for the indirect Immunofluorescence staining of fixed mammalian cells for plasma membrane and ER bound proteins

Cells are grown in FCS media on polylysine-coated 8 well chamber slides to 50% confluency. Then fixation of the cells is performed in 4% ParaFormAldehyde diluted in Phosphate Buffer Saline (PBS) solution (0.14M Phosphate, 0.1M NaCl pH 7.4). The cells are incubated for 30 minutes at room temperature in 300 microliters per well. Quenching is performed in 0.1M glycine in PBS for 2x 20 minutes at room temperature. Blocking is performed with 1% Bovine Serum Albumin (BSA) in 0.3 % saponin + PBS for at least 1 hour at room temperature. Incubation of the primary antibodies is performed in the blocking solution overnight at +4°C. The proper dilution of the antibodies is determined on a case-to-case basis. Cells are washed in PBS containing 0.3% Saponin for 2x 20 minutes at room temperature. Incubation of the secondary antibodies is performed in the blocking solution. Alexa 594 coupled goat anti-rabbit is diluted 1:1000 (Molecular Probes). Alexa 488 coupled goat anti-mouse is diluted 1:1000 (Molecular Probes). DAPI is used to label DNA. If phalloidin is used to label F-actin, the drug is diluted 1:500 and incubated with the secondary antibodies. Cells are then washed again 2x 20 minutes at room temperature in PBS. The excess of buffer is removed and cells are mounted in a media containing an anti-bleaching agent (Vectashield, Vector Laboratories).

### 3.2 Protocol for the indirect Immunofluorescence staining of fixed mammalian cells for non-plasma membrane bound proteins:

Cells are grown in FCS media on Polylysine coated 8 well chamber slides to 50% confluency. Fixation of the cells is performed in 4% ParaFormAldehyde diluted in Phosphate Buffer Saline (PBS) solution (0.14M Phosphate, 0.1M NaCl pH 7.4) for 30 minutes at Room Temperature (RT), 300 microliters per well. Quenching is performed in 0.1 M Glycine in PBS for 2x 20 minutes at roon temperature. Permeabilization of cells is done with 0.5% Triton X-100 in PBS for 10 minutes at room temperature. Blocking is then done in 1% Bovine Serum Albumin (BSA) in 0.3% Saponin + PBS for at least 1 hour at RT (Blocking solution). Incubation of the primary antibodies is performed in the blocking solution, overnight at +4°C. The proper dilution of the antibodies has to be determined on a case-to-case basis. Cells are washed in PBS containing 0.3% Saponin, for 2x 20 minutes at RT. Incubation of the secondary antibodies is performed in the blocking solution. Alexa 594 coupled goat anti-rabbit is diluted 1:1000 (Molecular Probes), Alexa 488 coupled goat anti-mouse is diluted 1:1000 (Molecular Probes). DAPI is used to label DNA. If Phalloidin is used to label F-actin, the drug is diluted 1:500 and incubated with the secondary antibodies. Cells are washed 2x 20 minutes at RT in PBS. The excess of buffer is removed and cells are mounted in a media containing an anti-bleaching agent (Vectashield, Vector Laboratories).

### 3.3 Immunoblot analysis

To analyze expression levels of TAP-tagged proteins, a cell pellet (from a 6-well dish) is lyzed in 60 µl DNAse I buffer (5% Glycerol, 100 mM NaCl, 0.8 % NP-40 (IGEPAL), 5 mM magnesium sulfate, 100 µg/ml DNAse I (Roche Diagnostics), 50 mM Tris, pH 7.5, protease inhibitor cocktail) for 15 min on ice. Each sample is split into two aliquots. The first half is centrifuged at 13,000 rpm for 5 min. to yield the NP-40-extractable material in the supernatant; the second half (total material) is carefully triturated. 50 pg each of the NP-40-extractable material and the total material are mixed with DTT-containing sample buffer for 30 min at 50°C on a shaker and separated by SDS polyacrylamide gel electrophoresis on a precast 4-12% Bis-Tris gel (Invitrogen). Proteins are then transferred to nitrocellulose using a semi-dry procedure with a discontinuous buffer system. Briefly, gel and nitrocellulose membrane are stacked between filter papers soaked in either anode buffer (three layers buffer A1 (0.3 M Tris-HCl) and three layers buffer A2 (0.03 M Tris-HCl)) or cathode buffer (three layers of 0.03 M Tris-HCl, pH 9.4, 0.1 % SDS, 40 mM epsilon a minocapronic acid). Electrotransfer of two gels at once is performed at 600 mA for 25 min. Transferred proteins are visualized with Ponceau S solution for one min to control transfer efficiency and then destained in water. The membrane is blocked in 5% non-fat milk powder in TBST (TBS containing 0.05% Tween-20) for 30 min at room temperature. It is subsequently incubated with HRP-coupled PAP antibody (1:5000 diluted in 5% milk/TBST) for 1 h at room temperature, washed three times for 10 min in TBST. The blot membrane is finally soaked in chemiluminescent substrate (ECL, Roche Diagnostics) for 2 min. and either exposed to X-ray film or analyzed on an imaging station.

### Part II: Cellular Eph kinase tyrosine phosphorylation assay

Cells are plated in 6-well plates in growth medium (see above). To test potential modulators, preferably inhibitors, of Eph kinase tyrosine phosphorylation, cells are starved in serum-free medium for 4 h. Under these conditions, Eph-TAP kinase is partially constitutively phophorylated-presumably through autophosphorylation that is commonly associated with over-expression of receptor tyrosine kinases. This feature is exploited to monitor the effect of Eph kinase modulators. Test compounds are diluted (from a stock in DMSO) in serum-free medium and added to cells for indicated times. Cells are chilled on ice and washed with ice-cold PBS. They are then lysed in 300 µl lysis buffer (50 mM Tris-HCl, 1% NP-40, 0.05% SDS, 100 mM NaCl, 1.5 mM MgCl2, 2 mM EDTA, 5% glycerol, pH 7.6) for 20 min at 4°C and cleared by centrifugation at 16,100xg in a table-top centrifuge for 20 min.
Lysates are subsequently incubated with 20 µl pre-washed rabbit IgG beads (Sigma) for 2 h with constant agitation at 4°C. Beads are finally washed with 1 ml of lysis buffer and immobilized protein complexes are eluted by boiling in 40 µl SDS sample buffer.
Samples are separate on a NuPAGE gradient gel (Invitrogen, 4-12%, 1.0 mm), transferred to PVDF membrane and studied by Western blotting using standard procedures. Blot membranes are probed with monoclonal antibody 4G10 (Upstate Biotechnology) or with HRP-coupled PAP antibody (see above).

### Example 3:

Modulation of APP processing/secretion by recombinant chimeric ephrin ligands or Eph receptor modulators

To induce clustering of recombinant ephrinB3/Fc (R&D systems) (Penzes et al., 2003. Neuron. 2003 Jan 23;37(2):263-74. Rapid induction of dendritic spine morphogenesis by trans-synaptic ephrinB-EphB receptor activation of the Rho-GEF kalirin.)10 µg of the chimeric protein are incubated with 1 µg/ml anti-human Fc antibody (Pierce) in OptiMEM for 30 min at RT.
SKNBE2 cells (or another suitable cell line) stably over-expressing human APP695 (SKNBE2/APP695) or a suitable mutant with enhanced beta-/gamma-secretase cleavage kinetics are serum-starved for 4 h. Pre-clustered ephrin or an Eph receptor modulator, preferably inhibitor, diluted in serum-free medium, is then added and incubated for suitable periods of time. Cell supernatants are collected and levels of Aβ1-42 determined by ELISA (Innogenetics).

### Example 4:

### Stimulation of APP processing/secretion by over-expression of Eph kinases or constitutively active mutants thereof

Eph kinase, preferably carrying an epitope tag such as the TAP tag, or constitutively active mutants thereof are stably expressed in SKNBE2 cells (or another suitable cell line) stably over-expressing human APP695 (SKNBE2/APP695) or a suitable mutant with enhanced beta-/gamma-secretase cleavage kinetics. Levels of Aβ1-42 secreted by parental and daughter cell lines are then compared at suitable points in time. Cell supernatants are collected and levels of Aβ1-42 determined by ELISA (Innogenetics).

### Example 5:

### Selective inhibition of EphB kinases by over-expression of ephrinB1 ligand "in cis"

Rationale: In-vivo Eph kinases and their ephrin ligands are typically expressed on neighboring cells (in "trans") and signaling is initiated by cell-to-cell contact. Yin et al. (2004) (Neurosci Res. 2004 Mar;48(3):285-96. EphA receptor tyrosine kinases interact with co-expressed ephrin-A ligands in cis.) have recently shown, however, that Eph kinases and ephrin ligands when co-expressed in the same cell (i.e. in "cis") interact via their functional binding domains, and that this interaction does not seem to mediate intracellular signals, but has an inhibitory effect on the trans interaction.

By super-transduction with different viral titres (see Example 2), SKNBE2/APP695 cell lines (or other suitable cell lines stably over-expressing wild-type APP or a suitable mutant thereof) are generated that stably express increasing levels of ephrinB1-TAP in "cis" with endogenous Eph kinases. Expression of the TAP-tagged protein is tested as outlined above. To examine the effect of over-expressing ephrinB1-TAP in "cis" on APP processing/Abeta secretion, equal numbers of cells are plated (7000 per 96-well) in growth medium. Serum-free medium is substituted for growth medium after 24 h and supernatants collected after another over night incubation. Levels of Aβ1-42 in supernatants are determined by ELISA (Innogenetics). To control for possible effects on cell viability, the MTT assay (Roche Diagnostics) is used according to manufacturer's recommendations.

### References:

Andres AC, Munarini N, Djonov V, Bruneau S, Zuercher G, Loercher S, Rohrbach V, Ziemiecki A (2003) EphB4 receptor tyrosine kinase transgenic mice develop glomerulopathies reminiscent of aglomerular vascular shunts. Mech Dev. 120(4):511-6.
Batlle E, Henderson JT, Beghtel H, van den Born MM, Sancho E, Huls G, Meeldijk J, Robertson J, van de Wetering M, Pawson T, Clevers H (2002) Beta-catenin and TCF mediate cell positioning in the intestinal epithelium by controlling the expression of EphB/ephrinB. Cell 111(2):251-63.
Bennett BD, Wang Z, Kuang WJ, Wang A, Groopman JE, Goeddel DV, Scadden DT (1994) Cloning and characterization of HTK, a novel transmembrane tyrosine kinase of the EPH subfamily. J Biol Chem. 269(19):14211-8.
Brantley DM, Cheng N, Thompson EJ, Lin Q, Brekken RA, Thorpe PE, Muraoka RS, Cerretti DP, Pozzi A, Jackson D, Lin C, Chen J (2002) Soluble Eph A receptors inhibit tumor angiogenesis and progression in vivo. Oncogene 21(46):7011-26.
Bundesen LQ, Scheel TA, Bregman BS, Kromer LF (2003) Ephrin-B2 and EphB2 regulation of astrocyte-meningeal fibroblast interactions in response to spinal cord lesions in adult rats. J Neurosci. 23(21):7789-800.
Chen ZY, Sun C, Reuhl K, Bergemann A, Henkemeyer M, Zhou R (2004) Abnormal hippocampal axon bundling in EphB receptor mutant mice. J Neurosci. 24(10):2366-74.
Contractor A, Rogers C, Maron C, Henkemeyer M, Swanson GT, Heinemann SF (2002) Trans-synaptic Eph receptor-ephrin signaling in hippocampal mossy fiber LTP. Science 296(5574):1864-9.
Dravis C, Yokoyama N, Chumley MJ, Cowan CA, Silvany RE, Shay J, Baker LA, Henkemeyer M (2004) Bidirectional signaling mediated by ephrin-B2 and EphB2 controls urorectal development. Dev Biol. 271(2):272-90.
Freywald A, Sharfe N, Roifman CM (2002) The kinase-null EphB6 receptor undergoes transphosphorylation in a complex with EphB1. J Biol Chem. 277(6):3823-8.
Gerety SS, Wang HU, Chen ZF, Anderson DJ (1999) Symmetrical mutant phenotypes of the receptor EphB4 and its specific transmembrane ligand ephrin-B2 in cardiovascular development. Mol. Cell. 4(3):403-14.
Grunwald IC, Korte M, Wolfer D, Wilkinson GA, Unsicker K, Lipp HP, Bonhoeffer T, Klein R (2001) Kinase-independent requirement of EphB2 receptors in hippocampal synaptic plasticity. Neuron 32(6):1027-40.
Hafner C, Bataille F, Meyer S, Becker B, Roesch A, Landthaler M, Vogt T (2003) Loss of EphB6 expression in metastatic melanoma. Int J Oncol. 23(6):1553-9.
Hafner C, Schmitz G, Meyer S, Bataille F, Hau P, Langmann T, Dietmaier W, Landthaler M, Vogt T (2004) Differential gene expression of Eph receptors and ephrins in benign human tissues and cancers. Clin Chem. 50(3):490-9.
Halford MM, Armes J, Buchert M, Meskenaite V, Grail D, Hibbs ML, Wilks AF, Farlie PG, Newgreen DF, Hovens CM, Stacker SA (2000) Ryk-deficient mice exhibit craniofacial defects associated with perturbed Eph receptor crosstalk. Nat Genet. 25(4):414-8.
Hattori M, Osterfield M, Flanagan JG (2000) Regulated cleavage of a contact-mediated axon repellent. Science 289(5483):1360-5.
Henderson JT, Georgiou J, Jia Z, Robertson J, Elowe S, Roder JC, Pawson T (2001) The receptor tyrosine kinase EphB2 regulates NMDA-dependent synaptic function. Neuron 32(6):1041-56.
Henkemeyer M, Orioli D, Henderson JT, Saxton TM, Roder J, Pawson T, Klein R (1996) Nuk controls pathfinding of commissural axons in the mammalian central nervous system. Cell 86(1):35-46.
Henkemeyer M, Itkis OS, Ngo M, Hickmott PW, Ethell IM (2003) Multiple EphB receptor tyrosine kinases shape dendritic spines in the hippocampus. J Cell Biol. 163(6):1313-26.
Himanen JP, Rajashankar KR, Lackmann M, Cowan CA, Henkemeyer M, Nikolov DB (2001) Crystal structure of an Eph receptor-ephrin complex. Nature 414(6866):933-8.
Himanen JP, Chumley MJ, Lackmann M, Li C, Barton WA, Jeffrey PD, Vearing C, Geleick D, Feldheim DA, Boyd AW, Henkemeyer M, Nikolov DB (2004) Repelling class discrimination: ephrin-A5 binds to and activates EphB2 receptor signaling. Nat Neurosci. 7(5):501-9.
Huusko P, Ponciano-Jackson D, Wolf M, Kiefer JA, Azorsa DO, Tuzmen S, Weaver D, Robbins C, et al. (2004) Nonsense-mediated decay microarray analysis identifies mutations of EPHB2 in human prostate cancer. Nat Genet. 36(9):979-83.
Ikegaki N, Tang XX, Liu XG, Biegel JA, Allen C, Yoshioka A, Sulman EP, Brodeur GM, Pleasure DE (1995) Molecular characterization and chromosomal localization of DRT (EPHT3): a developmentally regulated human protein-tyrosine kinase gene of the EPH family. Hum Mol Genet. 4(11):2033-45.
Kim I, Ryu YS, Kwak HJ, Ahn SY, Oh JL, Yancopoulos GD, Gale NW, Koh GY (2002) EphB ligand, ephrinB2, suppresses the VEGF- and angiopoietin 1-induced Ras/mitogen-activated protein kinase pathway in venous endothelial cells. FASEB J. 16(9):1126-8.
Koolpe M, Dail M, Pasquale EB (2002) An ephrin mimetic peptide that selectively targets the EphA2 receptor. J Biol Chem. 277(49):46974-9.
Lindberg RA, Hunter T (1990) cDNA cloning and characterization of eck, an epithelial cell receptor protein-tyrosine kinase in the eph/elk family of protein kinases. Mol Cell Biol. 10(12):6316-24.
Lu M, Miller KD, Gokmen-Polar Y, Jeng MH, Kinch MS (2003) EphA2 overexpression decreases estrogen dependence and tamoxifen sensitivity. Cancer Res. 63(12):3425-9.
Luo H, Yu G, Wu Y, Wu J (2002) EphB6 crosslinking results in costimulation of T cells. J Clin Invest. 110(8):1141-50.
Martiny-Baron G, Korff T, Schaffner F, Esser N, Eggstein S, Marme D, Augustin HG (2004) Inhibition of tumor growth and angiogenesis by soluble EphB4. Neoplasia. 2004 May-Jun;6(3):248-57.
Maru Y, Hirai H, Yoshida MC, Takaku F (1988) Evolution, expression, and chromosomal location of a novel receptor tyrosine kinase gene, eph. Mol Cell Biol. 8(9):3770-6.
Matsuoka H, Iwata N, Ito M, Shimoyama M, Nagata A, Chihara K, Takai S, Matsui T (1997) Expression of a kinase-defective Eph-like receptor in the normal human brain. Biochem Biophys Res Commun. 235(3):487-92.
Miao H, Wei BR, Peehl DM, Li Q, Alexandrou T, Schelling JR, Rhim JS, Sedor JR, Bumett E, Wang B (2001) Activation of EphA receptor tyrosine kinase inhibits the Ras/MAPK pathway. Nat Cell Biol. 3(5):527-30.
Miao H, Strebhardt K, Pasquale EB, Shen TL, Guan JL, Wang B (2004) Inhibition of integrin-mediated cell adhesion but not directional cell migration requires catalytic activity of EphB3 receptor tyrosine kinase: Role of Rho family small GTPases. J Biol Chem. 2004 Nov 9 [Epub ahead of print]
Nakada M, Niska JA, Miyamori H, McDonough WS, Wu J, Sato H, Berens ME (2004) The phosphorylation of EphB2 receptor regulates migration and invasion of human glioma cells. Cancer Res. 64(9):3179-85.
Pasquale EB (2004) Eph-ephrin promiscuity is now crystal clear. Nat. Neurosci. 7(5), 417.
Pratt RL, Kinch MS (2002) Activation of the EphA2 tyrosine kinase stimulates the MAP/ERK kinase signaling cascade. Oncogene 21(50):7690-9.
Prevost N, Woulfe D, Tanaka T, Brass LF (2002) Interactions between Eph kinases and ephrins provide a mechanism to support platelet aggregation once cell-to-cell contact has occurred. Proc Natl Acad Sci USA. 99(14):9219-24.
Purcell AL, Carew TJ (2003) Tyrosine kinases, synaptic plasticity and memory: insights from vertebrates and invertebrates. Trends Neurosci. 26(11):625-30.
Shimoyama M, Matsuoka H, Nagata A, Iwata N, Tamekane A, Okamura A, Gomyo H, Ito M, Jishage K, Kamada N, Suzuki H, Tetsuo Noda T, Matsui T (2002) Developmental expression of EphB6 in the thymus: lessons from EphB6 knockout mice. Biochem Biophys Res Commun. 298(1):87-94.
Slon-Usakiewicz JJ, Ng W, Foster JE, Dai JR, Deretey E, Toledo-Sherman L, Redden PR, Pasternak A, Reid N (2004) Frontal Affinity Chromatography with MS Detection of EphB2 Tyrosine Kinase Receptor. 1. Comparison with Conventional ELISA. J. Med. Chem. 47, 5094-5100.
Stephenson SA, Slomka S, Douglas EL, Hewett PJ, Hardingham JE (2001) Receptor protein tyrosine kinase EphB4 is up-regulated in colon cancer. BMC Mol Biol. 2(1):15.
Takasu MA, Dalva MB, Zigmond RE, Greenberg ME (2002) Modulation of NMDA receptor-dependent calcium influx and gene expression through EphB receptors. Science 295(5554):491-5.
Tang XX, Zhao H, Robinson ME, Cohen B, Cnaan A, London W, Cohn SL, Cheung NK, Brodeur GM, Evans AE, Ikegaki N (2000) Implications of EPHB6, EFNB2, and EFNB3 expressions in human neuroblastoma. Proc Natl Acad Sci USA. 97(20):10936-41.
Vindis C, Cerretti DP, Daniel TO, Huynh-Do U (2003) EphB1 recruits c-Src and p52Shc to activate MAPK/ERK and promote chemotaxis. J Cell Biol. 162(4):661-71.
Vindis C, Teli T, Cerretti DP, Turner CE, Huynh-Do U (2004) EphB1-mediated cell migration requires the phosphorylation of paxillin at Tyr-31/Tyr-118. J Biol Chem. 279(27):27965-70.
Wilkinson DG (2001) Multiple roles of EPH receptors and ephrins in neural development. Nat Rev Neurosci. 2(3):155-64.
Wissing J, Godl K, Brehmer D, Blencke S, Weber M, Habenberger P, Stein-Gerlach M, Missio A, Cotten M, Muller S, Daub H (2004) Chemical proteomic analysis reveals alternative modes of action for pyrido[2,3-d]pyrimidine kinase inhibitors. Mol Cell Proteomics Oct 8 [Epub ahead of print]
Wu Q, Suo Z, Risberg B, Karlsson MG, Villman K, Nesland JM (2004) Expression of Ephb2 and Ephb4 in breast carcinoma. Pathol Oncol Res. 10(1):26-33.
Wybenga-Groot, L. E.; Baskin, B.; Ong, S. H.; Tong, J.; Pawson, T.; Sicheri, F. (2001) Structural basis for autoinhibition of the EphB2 receptor tyrosine kinase by the unphosphorylated juxtamembrane region. Cell 106: 745-757.
Yin Y, Yamashita Y, Noda H, Okafuji T, Go MJ, Tanaka H (2004) EphA receptor tyrosine kinases interact with co-expressed ephrin-A ligands in cis. Neurosci Res. 48(3):285-96.

### Tables

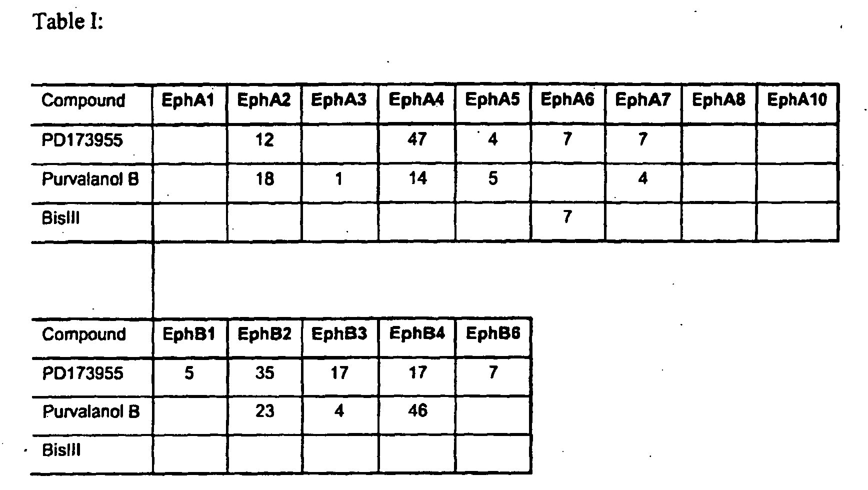

Eph kinases identified by LC-MS/MS in chemical proteomics experiments with three different compounds. The maximal number of non-redundant peptides identified for each Eph kinase in any such experiment is displayed.

## Claims

1. An in vitro method for the identification of a compound modulating, preferably inhibiting gamma secretase and/or beta secretase activity, comprising the steps of:
a) identifying an Eph receptor modulator, preferably an Eph receptor inhibitor, and
b) determining whether the Eph receptor modulator of step a) is capable of modulating, preferably inhibiting gamma secretase and/or beta secretase activity.
wherein in step a) the test compound is brought into contact with the Eph receptor and the interaction of the Eph receptor with the test compound is determined.

2. The method of claim 1, wherein in step a) the ability of the Eph receptor modulator to modulate Eph receptor kinase activity is measured.

3. The method of any of claims 1 or 2, wherein in step b) it is determined whether the Eph receptor modulator is capable of modulating, preferably lowering Abeta 42 production.

4. The method of any of claims 1 to 3, wherein the Eph receptor is an EphB receptor.

5. The method of claim 4, wherein the EphB receptor is selected from the group consisting of EphB1, EphB2, EphB3, EphB4, EphB5, and EphB6.

6. The method of any of claims 1 to 3, wherein the receptor is an EphA receptor.

7. The method of claim 6, wherein the EphA receptor is selected from the group consisting of EphA1, EphA2, EphA3, EphA4, EphA5, EphA6, EphA7, EphA8, EphA9, and EphA 10.

8. The method of any of claims 1 to 7, wherein the compound is selected from the group consisting of antibodies, chemically modified ephrins, peptidomimetics of ephrins, binding peptides, and low molecular weight molecules (LMWs), preferably organic small molecule drugs.

## Patentansprüche

1. In-vitro-Verfahren für die Identifizierung einer modulierenden Verbindung, vorzugsweise Gamma-Sekretase- und/oder Beta-Sekretaseaktivität inhibierend, umfassend die Schritte:
a) Identifizieren eines Eph-Rezeptormodulators, vorzugsweise eines Eph-Rezeptorinhibitors, und
b) Bestimmen, ob der Eph-Rezeptormodulator aus Schritt a) in der Lage ist, zu modulieren, vorzugsweise Gamma-Sekretase- und/oder Beta-Sekretaseaktivität zu inhibieren,
wobei in Schritt a) die Testverbindung mit dem Eph-Rezeptor in Kontakt gebracht wird, und die Interaktion des Eph-Rezeptors mit der Testverbindung bestimmt wird.

2. Verfahren nach Anspruch 1, wobei in Schritt a) die Fähigkeit des Eph-Rezeptormodulators, Eph-Rezeptorkinaseaktivität zu modulieren, gemessen wird.

3. Verfahren nach einem beliebigen der Ansprüche 1 oder 2, wobei in Schritt b) bestimmt wird, ob der Eph-Rezeptormodulator in der Lage ist, zu modulieren, vorzugsweise die Abeta-42-Produktion herabzusetzen.

4. Verfahren nach einem beliebigen der Ansprüche 1 bis 3, wobei der Eph-Rezeptor ein EphB-Rezeptor ist.

5. Verfahren nach Anspruch 4, wobei der EphB-Rezeptor ausgewählt ist aus der Gruppe bestehend aus EphB1, EphB2, EphB3, EphB4, EphB5 und EphB6.

6. Verfahren nach einem beliebigen der Ansprüche 1 bis 3, wobei der Rezeptor ein EphA-Rezeptor ist.

7. Verfahren nach Anspruch 6, wobei der EphA-Rezeptor ausgewählt ist aus der Gruppe bestehend aus EphA1, EphA2, EphA3, EphA4, EphA5, EphA6, EphA7, EphA8, EphA9 und EphA10.

8. Verfahren nach einem beliebigen der Ansprüche 1 bis 7, wobei die Verbindung ausgewählt ist aus der Gruppe bestehend aus Antikörpern, chemisch modifizierten Ephrinen, Peptidomimetika von Ephrinen, bindenden Peptiden und niedermolekularen Molekülen (low molecular weight molecules, LMWs), vorzugsweise organischen, niedermolekularen Wirkstoffen.

## Revendications

1. Méthode in vitro pour l'identification d'un composé modulant, de préférence inhibant l'activité de la gamma secretase et/ou de la beta secretase, comprenant les étapes :
a) d'identification d'un modulateur du récepteur Eph, de préférence un inhibiteur du récepteur Eph, et
b) de détermination si le modulateur du récepteur Eph de l'étape a) est capable de moduler, de préférence inhiber l'activité de la gamma secretase et/ou de la beta secretase,
dans laquelle on met en contact le composé de test dans l'étape a) avec le récepteur Eph et on détermine l'interaction du récepteur Eph avec le composé de test.

2. Méthode selon la revendication 1, dans laquelle on mesure dans l'étape a) la capacité du modulateur du récepteur Eph à moduler l'activité kinase du récepteur Eph.

3. Méthode selon l'une des revendications 1 ou 2, dans laquelle on détermine dans l'étape b) si le modulateur du récepteur Eph est capable de moduler, de préférence de diminuer la production d'Abeta 42.

4. Méthode selon l'une des revendications 1 à 3, dans laquelle le récepteur Eph est un récepteur EphB.

5. Méthode selon la revendication 4, dans laquelle le récepteur EphB est choisi dans le groupe consistant en EphB1, EphB2, EphB3, EphB4, EphB5 et EphB6.

6. Méthode selon l'une des revendications 1 à 3, dans laquelle le récepteur est un récepteur EphA.

7. Méthode selon la revendication 6, dans laquelle le récepteur EphA est choisi dans le groupe consistant en EphA1, EphA2, EphA3, EphA4, EphA5, EphA6, EphA7, EphA8, EphA9 et EphA10.

8. Méthode selon l'une quelconque des revendications 1 à 7, dans laquelle le composé est choisi dans le groupe consistant en des anticorps, des éphrines modifiées chimiquement, des éphrines de peptidomimétiques, des peptides liant, et des molécules de faible poids moléculaire (LMWs), de préférence des petites molécules médicamenteuses organiques.
